# EUROPEAN PATENT SPECIFICATION

(11) **EP 2 324 057 B1**
(45) Date of publication and mention of the grant of the patent: **21.03.2018**
(21) Application number: 09781618.5
(22) Date of filing: 07.08.2009
(51) Int. Cl.: C07K 14/705, C12N 15/62, A61K 39/00

(54) **MINIGENE COMPRISING HTPA SIGNAL PEPTIDE, T-CELL EPITOPES, E. COLI LTB AND FURIN SENSITIVE LINKERS**
HTPA-SIGNALPEPTID, T-ZELLEN-EPITOPE, E. COLI-LTB UND FURINEMPFINDLICHE LINKER UMFASSENDES MINIGEN
MINIGÈNE COMPORTANT LE PEPTIDE SIGNAL HTPA, DES ÉPITOPES DE LYMPHOCYTES T, LTB D'ESCHERICHIA COLI ET DES LIANTS SENSIBLES À LA FURINE

(30) Priority: 12.08.2008 US 188762 P
(43) Date of publication of application: 25.05.2011
(73) Proprietor: MSD Italia S.r.l., 00189 Rome (IT); Merck Sharp & Dohme Corp., Rahway, NJ 07065-0907 (US)
(72) Inventor: Aurisicchio, Luigi, I-1-00040 Pomezia (Rome) (IT); Bagchi, Ansuman, Rahway New Jersey 07065 (US); Ciliberto, Gennaro, I-1-00040 Pomezia (Rome) (IT); Fridman, Arthur, Rahway New Jersey 07065 (US); La Monica, Nicola, I-1-00040 Pomezia (Rome) (IT); Scarselli, Elisa, I-1-00040 Pomezia (Rome) (IT)
(74) Representative: Horgan, James Michael Frederic
(86) International application number: PCT/EP2009/060282
(87) International publication number: WO 2010/018136

(56) References cited:
- WO-A1-2008/043760
- ISHIOKA G Y ET AL: "UTILIZATION OF MHC CLASS I TRANSGENIC MICE FOR DEVELOPMENT OF MINIGENE DNA VACCINES ENCODING MULTIPLE HLA-RESTRICTED CTL EPITOPES" JOURNAL OF IMMUNOLOGY, AMERICAN ASSOCIATION OF IMMUNOLOGISTS, US, vol. 162, 1 January 1999 (1999-01-01), pages 3915-3925, XP002941189 ISSN: 0022-1767 cited in the application
- THOMSON S A ET AL: "DELIVERY OF MULTIPLE CD8 CYTOTOXIC T CELL EPITOPES BY DNA VACCINATION" JOURNAL OF IMMUNOLOGY, AMERICAN ASSOCIATION OF IMMUNOLOGISTS, US, vol. 160, 1 January 1998 (1998-01-01), pages 1717-1723, XP002939608 ISSN: 0022-1767 cited in the application
- LU JUN ET AL: "Multiepitope Trojan antigen peptide vaccines for the induction of antitumor CTL and Th immune responses." JOURNAL OF IMMUNOLOGY, vol. 172, no. 7, 1 April 2004 (2004-04-01) , pages 4575-4582, XP002558634 ISSN: 0022-1767 cited in the application
- LIPFORD G B ET AL: "Peptide engineering allows cytotoxic T-cell vaccination against human papilloma virus tumour antigen, E6" 1995, IMMUNOLOGY, VOL. 84, NR. 2, PAGE(S) 298-303 , XP002558635 ISSN: 0019-2805 abstract

## Description

### FIELD OF THE INVENTION

The present invention relates to a minigene suitable as a prophylactic or therapeutic vaccine against conditions such as cancer, infectious diseases or autoimmune diseases, pharmaceutical compositions comprising the minigene, and the use of the minigene in therapy.

### BACKGROUND OF THE INVENTION

It is recognised in the art (Ishioka et al, J. Immunol., 162, 3915-3925, 1999) that immunizations with minigenes containing T-cell epitopes may have several advantages compared to full-length proteins. Proteins may have unknown and potentially toxic biological activity while minigenes deliver only immunologically relevant genetic information. Immunization with a protein usually leads to an immune response that is narrowly focused on a few epitopes (Yewdell and Bennink, Annu. Rev. Immunol., 17, 51-88, 1999), while minigenes can induce significant immune response to multiple (up to 10 or more) epitopes simultaneously (Thomson et al, J. Immunol., 160, 1717-1723, 1998). Minigenes are short and compatible with commonly used delivery vectors including plasmid DNA and adenoviruses.

Minigenes are known in the art comprising HLA allele restricted T-cell epitopes, rather than the full sequence of the protein against which protection is required. Thus Thomson et al, *supra* discloses a minigene comprising multiple contiguous minimal murine CTL epitopes. Ishioka et al, *supra* describes a minigene encoding nine contiguous dominant HLA-A2.1- and A11-restricted epitopes from the polymerase, envelope and core proteins of hepatitis B virus and HIV, together with the PADRE (pan-DR epitope) universal T-cell epitope and an endoplasmic reticulum-translocating signal sequence.

The prior art also describes minigenes having spacers between the epitopes and other components. Thus Wang et al, Scand. J. Immunol., 60, 219-225, 2004 discloses a minigene having CTL epitopes of MPT64 and 38kDa proteins of Mycobacterium tuberculosis. Minigenes have been prepared with spacers having the sequence Ala-Ala-Tyr and/or ubiquitin functioning as a protein-targeting sequence. Pitcovski et al, Vaccine, 24, 636-643, 2006 describes a melanoma multiepitope polypeptide having three repeats of four modified melanoma antigens linked by five spacer elements that encode a signal for proteasomal cleavage, which polypeptide is fused to Escherichia coli heat labile enterotoxin (LTB). Zhan et al, J. Clin. Invest., 113, 1792-1798, 2004 discusses an oral DNA minigene vaccine containing the HIV tat translocation peptide, a spacer (AAA) and then an HLA-A2-restricted CEA T-cell epitope. The minigene was inserted into a pCMV vector comprising an ER signal peptide. Lu et al, J. Immunol., 172, 4575-4582, 2004, describes minigenes having multiple CTL epitopes joined via furin-sensitive linkers and also containing HIV-1 tat. WO 2008/043760 A1 discloses a minigene coding for a fusion protein comprising a TPA signal peptide followed by the complete hTERT protein as antigen and ended by a portion of LTB. The hTERT protein contains several T-cell epitopes. The minigene is used to treat or prevent cancer.

Despite extensive studies with the above minigenes, and the use of other strategies, the provision of an optimal minigene that maximizes epitope-specific immune responses remains elusive. To that end, the minigene provided by the present invention has been designed to maximise the expression and immunogenicity of each epitope in the minigene.

### DESCRIPTION OF DRAWINGS

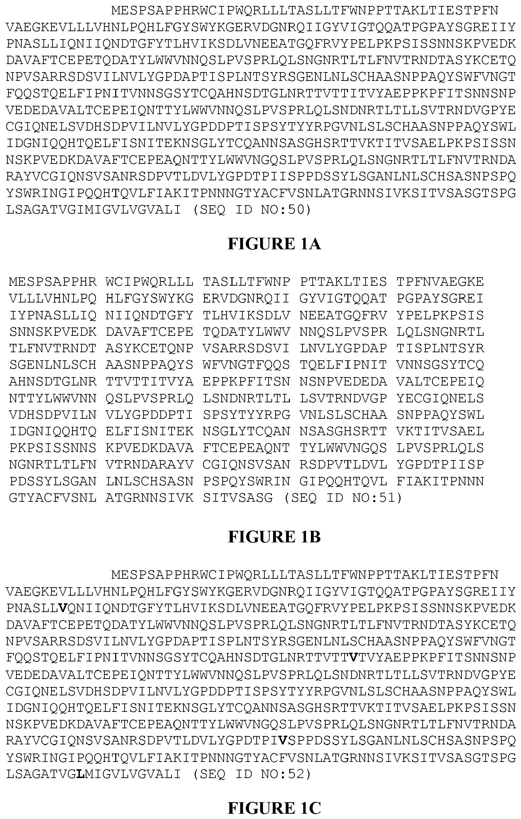

FIGURE 1, panel A, shows the amino acid sequence of the human CEA protein (SEQ ID NO:50), as set forth in NCBI Genbank Accession No. M17303. Panel B shows an exemplary CEA protein sequence which is deleted of its C-terminal anchoring domain (SEQ ID NO:51). Panel C shows an exemplary variant CEA protein sequence (SEQ ID NO:52) which comprises analogs SEQ ID NO:4, SEQ ID NO:5, SEQ ID NO:7, and SEQ ID NO:9. Modifications to the wt CEA sequence are shown in bold.
FIGURE 2 summarizes results obtained when comparing the top-scoring CEA-proteins to the human proteome. Of the 150 top-scoring CEA peptides, 45 had no matches in other human proteins, 64 matched a fragment of another CEA-like cell adhesion molecule, 26 matched a fragment of a pregnancy-specific glycoprotein, and 15 were similar to a fragment of a protein outside the CEA family. Altogether, 105/150 peptides were rejected or flagged.
FIGURE 3 shows the binding affinity of CEA epitope candidates. The stability of the peptide-MHC complex was evaluated by fluorescent activated cell sorting (FACS). The mean fluorescence intensity MFI resulting from the FACS analysis is shown for each epitope.
FIGURE 4 shows the relative binding stability of exemplary CEA epitopes described herein. The stability of peptide-MHC complex was evaluated by FACS analysis over time. MFI= mean fluorescence intensity.
FIGURE 5 shows *EI Suite*-selected peptides and analogs are immunogenic in HLA-A2.1 mice. HHD mice were immunized by subcutaneous injection of peptides. Two weeks later, cell mediated immune response was measured by ICS on pooled PBMCs.
FIGURE 6 shows analogs of immunogenic CEA peptides identified by *EI Suite* strongly increase immune reactivity against the corresponding wild type epitopes. HHD mice were immunized by SC injection of peptides. Two weeks later, cell mediated immune response against natural peptides was measured by ICS on individual mouse splenocytes.

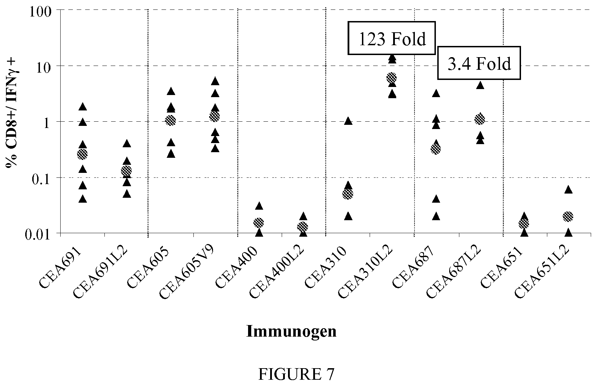

FIGURE 7 shows the immunogenicity of additional *EI Suite*-selected peptides (EXAMPLE 9). Groups of 6 HHD mice were vaccinated subcutaneously with 100µg of peptide in combination with HBVcore128 peptide (140 µg), IFA (1:1) and CpG (50µg). Mice received two injections two weeks apart and the immune response was measured by intracellular staining for IFNγ three weeks later. Each triangle represents the CMI of a single mouse; the geometric mean of each group is indicated by a round dot. Student's t-test between CEA310 and CEA310L2-vaccinated groups is statistically significant (p=0.007). The vaccination protocol has been repeated with similar results.
FIGURE 8 shows the binding affinity of hTERT candidate epitopes and analogues. The level of binding is expressed as a percent of positive control peptide binding. Wild type/analogue pairs have the same shading.
FIGURE 9 shows the relative binding stability of hTERT candidate epitopes and analogues. The y-axis shows the off-rate in hours.
FIGURE 10 shows that epitopes selected by *EI Suite*, and analogues, are immunogenic and that the analogues generate a stronger immune response than the wild type peptides. The responses of individual mice are shown as dots and the geometric mean is shown as a triangle.
FIGURE 11 shows the binding affinity of her-2/neu candidate epitopes and analogues. The level of binding is expressed as a percentage of positive control peptide binding. Wild type/analogue pairs have the same shading.
FIGURE 12 shows the relative binding stability of her-2/neu candidate epitopes and analogues. The γ-axis shows the off-rate in hours.
FIGURE 13 shows CMI response against wild type peptide in HHD mice immunized with her-2/neu peptides. The responses of individual mice are shown as dots and the geometric mean is shown as a triangle.
FIGURE 14 shows the general form of a minigene scaffold.
FIGURE 15 shows a minigene exemplifying the scaffold defined in this invention. TPA is human tissue plasminogen activator signal peptide; 411V10, 691, 589V10 and 682V10 are epitopes and immunogenic analogues from CEA; triangles are furin sensitive linker REKR; LTB is E. coli heat labile enterotoxin B subunit.
FIGURE 16 shows the modular structure of a minigene scaffold containing epitopes from one or more antigens restricted by multiple MHC alleles.
FIGURE 17 shows an alternative minigene scaffold containing epitopes from one or more antigens restricted by multiple MHC alleles.
FIGURES 18 and 19 show the CMI response against wild type peptides in HHD/CEA mice immunized with the minigene of Example 16. The responses of individual mice are shown in black; the group geometric mean is in grey.
FIGURE 20 shows a minigene containing universal helper peptide p30.
FIGURES 21 and 22 show the CMI response against wild type peptides in HHD/CEA mice immunized with the minigene construct of Example 20.
FIGURE 23 shows a minigene scaffold designed to evaluate dependence of immune response on epitope position.
FIGURE 24 shows the immune responses produced by the minigene shown in FIGURE 23 against target epitopes in HHD/CEA mice.
FIGURE 25 shows a ubiquitinized minigene construct containing one of the three spacer sequences AAY, LRA and RLRA.
FIGURE 26 shows the CMI responses produced by the minigene of FIGURE 25 in HHD/CEA mice in which the linker is (a) AAY, (b) LRA or (c) RLRA.
FIGURE 27 shows a minigene containing the HIV tat membrane-translocating sequence.
FIGURE 28 shows the CMI responses produced by the minigene of FIGURE 27 in HHD/CEA mice.

### DETAILED DESCRIPTION OF THE INVENTION

Thus the present invention provides a minigene coding for a protein comprising: (a) a human tissue plasminogen signal peptide; (b) at least one T-cell epitope; and (c) an E. coli heat labile enterotoxin B subunit; wherein (d) the at least one T-cell epitope is linked to the rest of the minigene, and to any other epitopes, by furin sensitive linkers having the sequence REKR.

Without being bound by theory, it is believed that the tissue plasminogen activator (TPA) signal peptide directs the minigene product to the endoplasmic reticulum (ER) where endoprotease furin cleaves the furin sensitive linkers to produce individual T-cell epitopes. Epitopes are then loaded onto empty major histocompatibility complex (MHC) molecules for export to the cell surface. Thus the TPA signal peptide enhances secretion of the translated product. TPA has the sequence MKRGLCCVLLLCGAVFVSPS (SEQ ID NO: 46).

Again, without being bound by theory, it is believed that the E-coli heat labile enterotoxin B (LTB) subunit of the secreted polypeptide binds to a receptor or on a professional antigen presenting cell (APC) resulting in polypeptide internalisation and APC maturation and activation. As a result an efficient T-cell response is induced. The LTB may be as described in WO-A-05077977 (IRBM). Thus it may be a truncated in its signal sequence. LTB has the amino acid sequence SRAPQSITELCSEYRNTQIYTINDKILSYTESMAGKREMVIITFKSGATFQVEVPGSQHIDS QKKAIERMKDTLRITYLTETKIDKLCVWNNKTPNSIAAISMEN (SEQ ID NO: 47) where SR at the N-terminus is an artificial linker.

In the present invention, the furin sensitive linkers have the sequence REKR. In an embodiment of the disclosure, the furin-sensitive linkers may have two or more different sequences.

The minigene of the invention may further comprise a T-helper epitope, such as tetanus toxin-derived universal helper peptide p2 or p30, see Valmori et al, J. Immunol., 149, 717-721, 1992.

In another embodiment the T-cell epitope is an immunogenic analogue of a naturally occurring epitope. Without being bound by theory, it is expected that analogues will form more stable complexes with MHC molecules compared to the corresponding wild-type epitopes (Lipford et al, Immunology, 84, 298-303, 1995). The longer half-life of analogue-MHC complexes on cell surfaces allows more efficient priming of T-cells which then recognise cognate wild-type epitopes on the surface of target cells (Keogh et al, J. Immunol., 167, 787-796, 2001). The minigenes of the invention may comprise solely wild-type epitopes, or solely analogues thereof, or a mixture of the two.

In an embodiment, the T-cell epitope is from a microbial or tumour associated antigen or from the variable domain of an autoantibody. The T-cell epitopes may be restricted by an HLA allele or the epitopes in the minigene may be restricted by more than one HLA allele. The T-cell epitopes may be restricted by HLA-A*0201. The T-cell epitope may be repeated one or more times in the minigene. Where the minigene contains epitopes restricted by more than one allele, the epitopes may be grouped by allelic restriction. Alternatively the epitopes, restricted by more than one allele, may be randomly distributed.

The minigene may comprise from two to twenty epitopes. It may comprise from 2 to 15, 2 to 10, 2 to 6, 4 to 20, 4 to 15, 4 to 10, 5 to 20, 5 to 15 or 5 to 20 epitopes. The minigene may comprise more than 20 epitopes.

The minigene may comprise T-cell epitopes from one or more of CEA, her-2/neu and hTERT.

The development of a minigene as a cancer vaccine capable of eliciting a clinically-relevant immune response is partly dependent on the choice of a target antigen that is preferentially expressed on tumor cells compared to normal cells.

Human CEA is one human associated antigen TAA that has been implicated in the pathogenesis of cancer. CEA is normally expressed during fetal development and in adult colonic mucosa. Aberrant CEA expression has long been correlated with many types of cancers, with the first report describing CEA overexpression in human colon tumors published over thirty years ago (Gold and Freedman, J. Exp. Med. 121:439-462 (1965)). Overexpression of CEA has since been detected in nearly all colorectal tumors, as well as in a high percentage of adenocarcinomas of the pancreas, liver, breast, ovary, cervix, and lung. Moreover, it was demonstrated in transgenic mice immunized with a recombinant *vaccinia* vector expressing CEA that anti-CEA immune responses could be elicited without inducing autoimmunity, making CEA a particularly attractive target for active and passive cancer immunotherapy (Kass et al. Cancer Res. 59: 676-83 (1999)).

Therapeutic strategies targeting CEA have included the use of CEA-based DNA and protein vaccines, and dendritic cell-based vaccines (for review, *see* Berinstein, *supra*; and Sarobe et al. Current Cancer Drug Targets 4: 443-54 (2004)). Antigenic peptide or epitope-based vaccines have also been investigated as a means of promoting the destruction of cancerous cells overexpressing CEA by an individual's immune system.

The ribonucleoprotein telomerase is expressed by more than 85% of human cancers, and therefore functions as a nearly universal TAA. Telomerase maintains the telomeric ends of linear chromosomes, protecting them from degradation and end-to-end fusion. Most human cells do not express telomerase and lose telomeric DNA with each cell division. In contrast, the great majority of human tumours exhibit strong telomerase activity, express human telomerase reverse transcriptase (hTERT) and maintain the length of their telomerases suggesting that the activation of telomerase plays an important role in the development of human cancers. The telomorase catalytic subunit hTERT is the rate-limiting component of the complex, and its expression correlates best with telomerase activity (Vonderheide et al, Immunity, 10, 673-679, 1999).

Therapeutic strategies targeting hTERT have included hTERT-based protein vaccines, and dendritic cell-based vaccines (Minev et al, PNAS, 97, 4796-4801, 2000; Vonderheide et al, Clin. Can. Res., 10, 828-839, 2004).

HER-2/neu is a transmembrane glycoprotein with tyrosine kinase activity whose structure is similar to epidermal growth factor. Amplification of her-2/neu gene and/or overexpression of the associated protein have been reported in many human adenocarcinomas of the breast, ovary, uterus, prostate, stomach, oesophagus, pancreas, kidney and lung.

Therapeutic strategies targeting her-2/neu have included CTLs from healthy donor PBMCs stimulated with HLA-A*0201-restricted epitopes and dendritic cell-based vaccines and an immunogenic protein with granulocyte-macrophage colony-stimulating factor (Scardino et al, J. Immunol., 168, 5900-5906, 2002; Brossart et al, Blood, 96, 3102-3108, 2000; Peoples et al, J. Clin. Oncol., 23, 7536-7545, 2005).

Immunogenic epitopes of hTERT and her-2/neu are described in Minev et al, *supra,* Keogh et al, J. Immunol., 167, 787-796, 2001 and Kono et al, Int. J. Cancer, 78, 202-208, 1998.

The T-lymphocyte cellular-mediated immune response forms a critical component of the immune response and plays a crucial role in the eradication of tumor cells by the mammalian immune system. T cell-mediated immune responses require the activation of cytotoxic (CD8+) and helper (CD4+) T lymphocytes. Cytotoxic T lymphocytes (CTL) and their T-cell receptors (TCR) recognize small peptides presented by major histocompatibility complex (MHC) class I molecules on the cell surface (Bjorkman P J., Cell 89:167-170 (1997); Garcia et al., Science 274:209-219 (1996)). The peptides are derived from intracellular antigens via the endogenous antigen processing and presentation pathway (Germain R N., Cell 76:287-299 (1994); Pamer et al., Annu Rev Immunol 16:323-358(1998)). Peptides for human CD8+ epitopes range from 7 to 14 amino acids, and typically are 9-10 amino acids in length. TCR recognition of the peptide-MHC class I molecule complexes on the cell surface triggers the cytolytic activity of CTL, resulting in the death of cells presenting the peptide-MHC class I complexes (Kagi et al., Science 265: 528-530 (1994)). MHC class I restricted epitope vaccines have been shown to confer protection in some animal models. The development of epitope vaccines encoding human HLA-restricted CTL epitopes capable of conferring broad, effective, and non-ethnically biased population coverage is highly desirable.

Epitope-based vaccines offer a number of advantageous features compared to vaccines based on full-length TAAs, including ease and low cost of peptide synthesis. In addition, peptide vaccines can induce immune responses to subdominant epitopes when there is tolerance to a dominant epitope, and anchor-modified or heteroclitic peptide analogs can be constructed that can break tolerance and/or further increase immunogenicity relative to native peptides (for review, *see* Lazoura and Apostolopoulos, Current Medicinal Chemistry 12: 1481-94 (2005)). The use of peptides as immunogens also minimizes safety risks associated with the use of intact proteins.

The identification of novel CEA epitopes and epitope analogs that generate effective antitumor immune responses without causing autoimmunity will allow the development of epitope-based cancer vaccines that elicit a clinically relevant prophylactic and/or therapeutic immune response against CEA.

The present invention thus includes the use of immunogenic peptides ("epitopes") of CEA, hTERT and her-2/neu and analogs thereof; which are selected based on their binding affinity for a Class I MHC allele, specifically, HLA-A*0201. The peptides and analogs described herein were selected based on their ability to elicit a maximum tumor-specific immune response in a tolerized setting, as well as for their minimal potential for eliciting off-target autoimmune activity. More specifically, the CEA, hTERT and her-2/neu proteins were scanned using a proprietary software package called *EI Suite* that ranks protein fragments based on binding affinity for HLA-A*0201, similarity to fragments of other human proteins, and amenability to immunogenic enhancement.

Wild-type CEA epitopes of use in the present invention include the sequence of amino acids shown in SEQ ID NO:1 or SEQ ID NO:7. Wild-type hTERT epitopes of use in the present invention may be selected from SEQ ID NO:13, SEQ ID NO:14, SEQ ID NO:16, SEQ ID NO:18, SEQ ID NO:19, SEQ ID NO:21, SEQ ID NO:23 and SEQ ID NO:24. Wild-type her-2/neu epitopes of use in the present invention may be selected from SEQ ID NO:25, SEQ ID NO:27, SEQ ID NO:28, SEQ ID NO:30, SEQ ID NO:32, SEQ ID NO:34, SEQ ID NO:36, SEQ ID NO:38, SEQ ID NO:40, SEQ ID NO:41, SEQ ID NO:43 and SEQ ID NO:44. One favoured epitope for her-2/neu is SEQ ID NO:36.

In addition to wild-type CEA, hTERT and her-2/neu epitopes, in some embodiments of the invention described herein, modifications were introduced at specific amino acid positions within the naturally occurring sequence of the corresponding wild-type immunogenic CEA, hTERT and her-2/neu peptides to form anchor-modified analogs. Said analogs may provide an increased benefit as a vaccine component if they induce an immune response cross-reactive against CEA which is superior in quality to that induced by the corresponding wild-type epitope. The immunogenic peptide analogs of the present invention comprise a sequence of amino acids selected from SEQ ID NO:3, SEQ ID NO:4, SEQ ID NO:5, SEQ ID NO:6, SEQ ID NO:8, SEQ ID NO:9, SEQ ID NO:10, SEQ ID NO:11, and SEQ ID NO:12 for CEA; SEQ ID NO:15, SEQ ID NO:17, SEQ ID NO:20 and SEQ ID NO:22 for hTERT; and SEQ ID NO:26, SEQ ID NO:29, SEQ ID NO:31, SEQ ID NO:33, SEQ ID NO:35, SEQ ID NO:37, SEQ ID NO:39, SEQ ID NO:42 and SEQ ID NO:45 for her-2/neu.

Also disclosed are polynucleotides encoding said immunogenic CEA, hTERT and her-2/neu peptides, peptide analogs, and variant CEA, hTERT or her-2/neu proteins, as well as recombinant expression vectors, including but not limited to, adenovirus and plasmid vectors, comprising said polynucleotides. In some embodiments, the vector is an adenovirus vector, which, in preferred embodiments, is selected from the group consisting of: Ad5, Ad6, and Ad24. In further exemplary embodiments, the polynucleotides comprise a sequence of nucleotides that is operably linked to a promoter. Also provided are recombinant host cells comprising the expression vectors described herein.

Further disclosed are pharmaceutical compositions comprising one or more of the immunogenic CEA, hTERT or her-2/neu peptides, analogs, variant CEA, hTERT or her-2/neu proteins, or nucleic acids encoding said peptides, analogs, and proteins, together with a pharmaceutically acceptable carrier. In preferred embodiments, the pharmaceutical composition comprises a plurality of immunogenic peptides or analogs thereof. In some embodiments, the pharmaceutical composition further comprises an adjuvant.

Also disclosed are vaccine compositions comprising one or more of the CEA, hTERT or her-2/neu peptide epitopes, analogs, variant proteins or comprising one or more polynucleotides encoding said epitopes, analogs, or variant proteins disclosed throughout the specification. In preferred embodiments, the vaccine compositions comprise a plurality of isolated polynucleotides, encoded peptides, or analogs thereof.

Further disclosed is a method of eliciting an immune response to CEA, hTERT or her-2/neu in a patient in need thereof, said method comprising introducing into the patient the pharmaceutical compositions or vaccines disclosed herein.

The present invention further provides methods for inhibiting the development of a cancer in a mammal, or treating or minimizing an existing cancer, by eliciting an immune response to CEA, hTERT or her-2/neu, such methods comprising administering a vaccine or pharmaceutical composition comprising one or more immunogenic CEA, hTERT or her-2/neu peptide described herein, or analog thereof, or polynucleotide encoding said peptide or analog, as described herein. In preferred embodiments of the methods herein, the immune response is enhanced relative to the response elicited by a wild-type CEA, hTERT or her-2/neu peptide.

As used throughout the specification, the following definitions and abbreviations apply:
The term "promoter" refers to a recognition site on a DNA strand to which the RNA polymerase binds. The promoter forms an initiation complex with RNA polymerase to initiate and drive transcriptional activity. The complex can be modified by activating sequences termed "enhancers" or inhibiting sequences termed "silencers".

The term "cassette" refers to a nucleotide or gene sequence that is to be expressed from a vector, for example, a nucleotide or gene sequence encoding one or more of the CEA peptide epitopes, analogs, or modified CEA proteins described herein. In general, a cassette comprises a gene sequence that can be inserted into a vector, which in some embodiments, provides regulatory sequences for expressing the nucleotide or gene sequence. In other embodiments, the nucleotide or gene sequence provides the regulatory sequences for its expression. In further embodiments, the vector provides some regulatory sequences and the nucleotide or gene sequence provides other regulatory sequences. For example, the vector can provide a promoter for transcribing the nucleotide or gene sequence and the nucleotide or gene sequence provides a transcription termination sequence. The regulatory sequences that can be provided by the vector include, but are not limited to, enhancers, transcription termination sequences, splice acceptor and donor sequences, introns, ribosome binding sequences, and poly(A) addition sequences.

The term "vector" refers to some means by which DNA fragments can be introduced into a host organism or host tissue. There are various types of vectors including plasmid, virus (including adenovirus), bacteriophages and cosmids.

The term "first generation," as used in reference to adenoviral vectors, describes adenoviral vectors that are replication-defective. First generation adenovirus vectors typically have a deleted or inactivated E1 gene region, and preferably have a deleted or inactivated E3 gene region.

The term "treatment" refers to both therapeutic treatment and prophylactic or preventative measures. Those in need of treatment include those already with the disorder as well as those prone to have the disorder or those in which the disorder is to be prevented.

A "disorder" is any condition that would benefit from treatment with the molecules of the present invention, including the CEA peptide epitopes, CEA epitope analogs, modified CEA proteins and nucleic acid molecules encoding said epitopes, analogs, and modified proteins. Encompassed by the term "disorder" are chronic and acute disorders or diseases including those pathological conditions which predispose the mammal to the disorder in question. The molecules of the present invention are intended for use as treatments for disorders or conditions characterized by aberrant cell proliferation, including, but not limited to, pancreatic cancer, liver cancer, breast cancer, colorectal cancer, ovarian cancer, cervical cancer, and lung cancer.

The term "effective amount" means sufficient vaccine composition is introduced to produce the adequate levels of the polypeptide, so that a clinically significant immune response results. One skilled in the art recognizes that this level may vary.

The term "nucleic acid" or "nucleic acid molecule" is intended for ribonucleic acid (RNA) or deoxyribonucleic acid (DNA), probes, oligonucleotides, fragment or portions thereof, and primers.

"Wild-type CEA", "wild-type hTERT", "wild-type her-2/neu" or "wild-type protein" or "wt protein" refers to a CEA protein comprising a naturally occurring sequence of amino acids, which sequence is encoded by the major allele of CEA found in the human population, free of induced mutations or modifications.

The term "variant protein", "variant CEA", "variant hTERT" or "variant her-2/neu" refers to a CEA, hTERT or her-2/neu protein comprising modifications to at least one specific amino acid residue of the CEA protein relative to the full-length wild-type CEA, hTERT or her-2/neu protein.

The term "mammalian" refers to any mammal, including a human being.

The abbreviation "Ag" refers to an antigen. The term "antigen" refers to any biologic or macromolecular substance that can be recognized by a T-cell or an antibody molecule.

The abbreviations "Ab" and "mAb" refer to an antibody and a monoclonal antibody, respectively.

The terms "major histocompatibility complex (MHC)" and "human leukocyte antigen (HLA)" are used interchangeably to refer to a locus of genes that encode proteins which present a vast variety of peptides onto the cell surface for specific recognition by a T-cell receptor. A subclass of MHC genes, called Class I MHC molecules, present peptides to CD8⁺ T-cells.

"Epitope" refers to a peptide which is a portion of an antigen, wherein the peptide comprises an amino acid sequence that is capable of stimulating an immune response. The MHC class I epitopes disclosed herein are useful in pharmaceutical compositions (e.g., vaccines) for stimulating an immune response directed to CEA. In preferred embodiments, epitopes according to this definition represent peptides which are likely to be non-covalently bound to the binding cleft of a class I MHC molecule on the surface of antigen presenting cells in a manner which facilitates its interaction with T-cell receptors (TCR). The term "epitope" is used interchangeably herein with the term "immunogenic peptide."

The term "wild type epitope" refers to an epitope comprising a sequence of nine or ten amino acids that can be found in the naturally occurring wild-type CEA protein as set forth in SEQ ID NO:1.

The terms "9-mer" and "10-mer" refer to a linear sequence of nine or ten amino acids that occur in a target antigen. It is generally understood that a collection of sequences that includes all possible 9-mers and 10-mers present in a parent sequence, comprise sequences which overlap by eight or nine residues, respectively.

The term "anchor residues" refers to the amino acid residues of an immunogenic peptide fragment that provide a contact point between the peptide and the MHC molecule. The anchor residues comprise side chains that fit into the peptide-binding clefts of said MHC molecules.

"Binding motif' refers to a specific pattern or combination of anchor residues within protein sequences which are correlated with the ability to bind to a specified HLA allele or serotypes.

"Immunogen" refers to specific antigens that are capable of inducing or stimulating an immune response. Not all antigens are immunogenic.

"Enhanced immunogenicity" refers an increased ability to activate the immune system when compared to the immune response elicited by the wild-type peptide. A variant peptide or analog can be said to have "increased immunogenicity" if it induces a higher level of T-cell activation relative to the level of activation induced by the corresponding wild-type peptide as measured in a standard *in-vitro* T-cell activation assay. In a preferred embodiment, the frequency of vaccination-induced epitope-specific T-cells will be increased at least 10-fold by the administration of an immunoenhanced analogs relative to the level of T-cell activation (i.e., number of epitope-specific CTLs) induced by immunization with the parent peptide. A 50-fold increase in T-cell activity is an especially preferred level of immunoenhancement.

"Immunogenic analog" or "epitope analog" refers to a peptide epitope with one or more residues of the wild-type amino acid sequence substituted with an alternative amino acid sequence identified by the immunoenhancement filter of *EI Suite*. Coordinated substitutions are often carried out to regulate or modify (e.g., increase) immunogenicity of a natural peptide.

The terms "prediction" and "predicting" are used herein refer to the use of the present teachings to estimate properties (e.g., ability to bind to MHC class I allele, likelihood of being efficiently processed and presented by APC, uniqueness to target antigen, immunogenicity) of amino acid sequences representing putative T-cell epitopes.

The terms "MHC class I binder" and "MHC peptide" are used to refer to peptides having a high known or predicted binding affinity for a mammalian class I major histocompatibility complex (MHC) molecule.

"Immunogenic composition" refers to a composition that is capable of inducing an immune response, a reaction, an effect, and/or an event. In some embodiments, such responses, reactions, effects, and/or events can be induced *in vitro* or *in vivo*. For example, the induction, activation, or expansion of cells involved in cell mediated immunity, such as CTLs represents an immune response, effect or an event. Representative immunogenic compositions include an immunoenhanced full-length target antigen or a minigene vaccine.

"Vaccine" refers to an immunogenic composition that is capable of eliciting a clinically relevant prophylactic and/or therapeutic immune response that prevents, cures, or ameliorates disease.

"Epitope vaccine" generally refers to a composition of several epitopes derived from one or more target proteins of the same, or different pathogen or tumor cell, specific to one or more alleles of interest. The list of epitopes used may include those optimized for natural processing, immunogenicity, uniqueness (e.g., lack of similarity to other self-antigens), population coverage, and predicted disease relevance. For example, an immunogenic composition comprising more than one putative T-cell epitope derived from at least one target antigen linked together, with or without additional amino acids ("spacers") between the epitopes can be used as an epitope vaccine. Thus, an epitope vaccine can stimulate immune responses directed to single or multiple epitopes of one or more antigens.

"*EI Suite*" refers to a software package useful for identifying immunogenic epitopes described in WO-A-06124408.

The CEA, hTERT and her-2/neu epitopes and analogs provided herein were selected based on their binding affinity for a class I MHC allele, specifically, HLA-A*0201. Said peptides and analogs were additionally selected based on their ability to elicit a maximum tumor-specific immune response in a tolerized setting, as well as for their minimal potential for eliciting off-target autoimmune activity.

More specifically, the CEA, hTERT and her-2/neu epitopes and analogs were initially selected by scanning the CEA, hTERT and her-2/neu protein sequences using a proprietary software package called *EI Suite* that ranks protein fragments based on binding affinity for a Class I MHC allele, in this case HLA-A*0201, similarity to fragments of other human and murine proteins, and amenability to immunogenic enhancement (*see* WO 2006/0257944). Epitopes and analogs predicted to be MHC class I binders were analyzed *in vitro* for binding affinity to T2 cells, which are HLA-A*0201 positive, MHC class II negative and TAP deficient. Moreover, the immunogenicity of the selected peptide epitopes and analogs was determined in HHD transgenic mice. HHD mice are transgenic for the HHD complex (human β2-microglobulin fused to HLA-A2.1 α1 and α2 domain, H-2D^{b} α3 domain) and are devoid of H-2D^{b} and murine β2-microglobulin. (Pascolo et al. J. Exp. Med. 185(12):2043-51 (1997)). For this reason, the immune response elicited in these mice is specifically restricted to human HLA-A2.1, making this line a suitable model for epitope identification and optimization.

The isolated immunogenic CEA, hTERT and her-2/neu peptides were predicted to be optimal vaccine candidates by *EI Suite* and were determined to bind HLA-A*0201 and to be immunogenic in HHD transgenic mice. CEA, hTERT and her-2/neu epitopes present in the major wild-type CEA allele and identified in this manner are disclosed in SEQ ID NOs: 1, 7, 13, 14, 16, 18, 19, 21, 23, 24, 25, 27, 28, 30, 32, 34, 36, 38, 40, 41, 43 and 44.

Class I MHC molecules are heterodimers of non-covalently bound MHC-encoded heavy (or alpha) chain, and a non-MHC-encoded β2-microglobulin light chain. There are four separate regions: 1) the peptide binding region, 2) the immunoglobulin-like region, 3) the transmembrane region and 4) a cytoplasmic region. The peptide-binding region is a groove which functions to accommodate a peptide ligand of 8-10 amino acid residues. To this end, the CEA, hTERT and her-2/neu epitopes and analogs consist of a linear sequence of nine or ten amino acids. In preferred embodiments, the epitopes and analogs are nine or ten amino acids in length.

The binding of peptide ligands to the MHC binding groove is specific and is stabilized at both ends by contacts between atoms in the free amino and carboxyl termini of the peptide and invariant sites that are found at each end of the cleft of all MHC class I molecules. Because the amino acid side chains at these positions insert into pockets in the MHC molecule and function to anchor the peptide to the MHC molecule they are commonly referred to as anchor residues. The bound peptide lies in an extended conformation along the groove. Anchor residues can be divided into primary and secondary. Primary anchor positions exhibit strong preferences for relatively well-defined sets of amino acid residues. Secondary positions show weaker and/or less well-defined preferences that can often be better described in terms of less favored, rather than more favored residues.

The anchor residues confer sequence selectivity and binding specificity to the interaction between the peptide ligand and the MHC molecule. The main anchor residues of human HLA class I molecules occur at positions 2 and the C-terminus of the peptides. Generally speaking, peptide-binding to a particular MHC molecule requires the peptide to have one or more specific amino acids at a fixed position, frequently the terminal or penultimate amino acid of the peptide. Since more stable binding will generally improve immunogenicity, anchor residues are preferably conserved or optimized in the design of analogs, regardless of their position.

As discussed above, it is known in the art that HLA class I binders modified at anchor positions (position 2 and the C-terminus position of the peptide), are often more immunogenic than the wild-type peptide due to improved binding to the HLA molecule (G. Lipford et. al., Immunology 84: 298-303 (1995)). At the same time, T-cells specific for the modified peptide generally also recognize the wild-type peptide, since the mutations are restricted to residues that do not make contact with the T-cell receptor. Based on this knowledge, the immunogenicity enhancement filter of *EI Suite* was utilized to identify anchor-modified analogs that comprise substitutions/mutations that optimize peptide/MHC binding interactions at the anchor positions. Said immunoenhancement filter identified substitutions within anchor residues of CEA T-cell epitope candidates to improve their immunogenicity. Immunoenhanced peptide analogs that are cross-reactive to the target antigen are beneficial for use in cancer vaccines targeting tumor-associated antigens to overcome tolerance and poor immunogenicity.

To this end, there is provided anchor-modified peptide analogs which can elicit an immune response against CEA, hTERT or her-2/neu that is stronger than the immune response elicited by the corresponding wild-type epitope. The peptide analogs of the present invention comprise a sequence of amino acids selected from those shown in Tables 1, 2 and 3. Said peptide analogs consist of a linear sequence of nine or ten amino acids.

Also disclosed are nucleotides encoding the immunogenic epitopes and epitope analogs which are useful either alone or in combination to construct DNA-based vaccines and minigenes targeting CEA, hTERT or her-2/neu. Said nucleotides are useful in genetic vaccines to elicit or enhance immunity to the protein product expressed by the CEA, hTERT and her-2/neu tumor-associated antigens.

Accordingly, the present invention relates to an isolated nucleic acid molecule or polynucleotide comprising a sequence of nucleotides encoding an epitope analog, said analog comprising a sequence of amino acids as set forth in Tables 1, 2 and 3. The nucleic acid molecules of the present invention are substantially free from other nucleic acids.

Also disclosed are recombinant vectors and recombinant host cells, both prokaryotic and eukaryotic, which contain the nucleic acid molecules disclosed throughout this specification. The isolated DNA molecules, associated vectors, and hosts of the present invention are useful for the development of a cancer vaccine.

Vectors may be comprised of DNA or RNA. For most cloning purposes, DNA vectors are preferred. Typical vectors include plasmids, modified viruses, baculovirus, bacteriophage, cosmids, yeast artificial chromosomes, and other forms of episomal or integrated DNA that can encode a CEA, hTERT or her-2/neu fusion protein. It is well within the purview of the skilled artisan to determine an appropriate vector for a particular gene transfer or other use.

An expression vector containing a CEA, hTERT or her-2/neu peptide-encoding nucleic acid molecule may be used for high-level expression of CEA, hTERT or her-2/neu peptides in a recombinant host cell. Expression vectors may include, but are not limited to, cloning vectors, modified cloning vectors, specifically designed plasmids or viruses. Also, a variety of bacterial expression vectors may be used to express recombinant CEA, hTERT or her-2/neu peptide sequences in bacterial cells if desired. In addition, a variety of fungal cell expression vectors may be used to express recombinant CEA, hTERT or her-2/neu peptide sequences in fungal cells. Further, a variety of insect cell expression vectors may be used to express recombinant peptides in insect cells.

Also disclosed are host cells transformed or transfected with vectors comprising the nucleic acid molecules of the present invention. Recombinant host cells may be prokaryotic or eukaryotic, including but not limited to, bacteria such as *E. coli*, fungal cells such as yeast, mammalian cells including, but not limited to, cell lines of bovine, porcine, monkey and rodent origin; and insect cells including but not limited to *Drosophila* and silkworm derived cell lines. In one embodiment of the invention, the host cell is a yeast cell which is selected from the group consisting of: *Saccharomyces cerevisiae, Hansenula polymorpha, Pichia pastoris, Kluyvermyces fragilis, Kluveromyces lactis,* and *Schizosaccharomyces pombe*. Such recombinant host cells can be cultured under suitable conditions to produce a CEA, hTERT or her-2/neu peptide epitope or epitope analog. In one embodiment of the present invention, the host cell is human. As defined herein, the term "host cell" is not intended to include a host cell in the body of a transgenic human being, human fetus, or human embryo.

The nucleic acids may be assembled into an expression cassette which comprises sequences designed to provide for efficient expression of the peptide, analog, variant protein, or minigene in a human cell. The cassette preferably contains a peptide epitope or analog-encoding gene, or minigene, with related transcriptional and translations control sequences operatively linked to it, such as a promoter, and termination sequences. The promoter may be the cytomegalovirus promoter without the intron A sequence (CMV), although those skilled in the art will recognize that any of a number of other known promoters, or other eukaryotic gene promoters may be used. A preferred transcriptional terminator is the bovine growth hormone terminator, although other known transcriptional terminators may also be used. The combination of CMV-BGH terminator is particularly preferred.

The CEA, hTERT or her-2/neu peptide expression cassette is inserted into a vector. The vector is preferably an adenoviral or plasmid vector, although linear DNA linked to a promoter, or other vectors, such as adeno-associated virus or a modified vaccinia virus, retroviral or lentiviral vector may also be used.

In one embodiment of the invention, the vector is an adenovirus vector (used interchangeably herein with "adenovector"). Adenovectors can be based on different adenovirus serotypes such as those found in humans or animals. Examples of animal adenoviruses include bovine, porcine, chimp, murine, canine, and avian (CELO). Preferred adenovectors are based on human serotypes, more preferably Group B, C, or D serotypes. Examples of human adenovirus Group B, C, D, or E serotypes include types 2 ("Ad2"), 4 ("Ad4"), 5 ("Ad5"), 6 ("Ad6"), 24 ("Ad24"), 26 ("Ad26"), 34 ("Ad34") and 35 ("Ad35"). In particularly preferred embodiments of the present invention, the expression vector is an adenovirus type 5 or 6 (Ad 5 or Ad6) vector.

If the vector chosen is an adenovirus, it is preferred that the vector be a so-called first-generation adenoviral vector. These adenoviral vectors are characterized by having a non-functional E1 gene region, and preferably a deleted adenoviral E1 gene region. Adenovectors do not need to have their E1 and E3 regions completely removed. Rather, a sufficient amount the E1 region is removed to render the vector replication incompetent in the absence of the E1 proteins being supplied *in trans*; and the E1 deletion or the combination of the E1 and E3 deletions are sufficiently large enough to accommodate a gene expression cassette.

In some embodiments, the expression cassette is inserted in the position where the adenoviral E1 gene is normally located. In addition, these vectors optionally have a non-functional or deleted E3 region. In some embodiments of the invention, the adenovirus genome used is deleted of both the E1 and E3 regions (ΔE1ΔE3). The adenoviruses can be multiplied in known cell lines which express the viral E1 gene, such as 293 cells, or PERC.6 cells, or in cell lines derived from 293 or PERC.6 cell which are transiently or stablily transformed to express an extra protein. For examples, when using constructs that have a controlled gene expression, such as a tetracycline regulatable promoter system, the cell line may express components involved in the regulatory system. One example of such a cell line is T-Rex-293; others are known in the art.

For convenience in manipulating the adenoviral vector, the adenovirus may be in a shuttle plasmid form. This invention is also directed to a shuttle plasmid vector which comprises a plasmid portion and an adenovirus portion, the adenovirus portion comprising an adenoviral genome which has a deleted E1 and optional E3 deletion, and has an inserted expression cassette comprising an epitope, analog, or CEA minigene. In preferred embodiments, there is a restriction site flanking the adenoviral portion of the plasmid so that the adenoviral vector can easily be removed. The shuttle plasmid may be replicated in prokaryotic cells or eukaryotic cells.

In one embodiment, the expression cassette is inserted into an Ad6 (ΔE1ΔE3) adenovirus plasmid (*See* Emini et al., WO2003031588A2). This vector comprises an Ad6 adenoviral genome deleted of the E1 and E3 regions. In some embodiments of the invention, the expression cassette is inserted into the pMRKAd5-HV0 adenovirus plasmid (*See* Emini et al., WO 02/22080). This plasmid comprises an Ad5 adenoviral genome deleted of the E1 and E3 regions. The design of the pMRKAd5-HV0 plasmid was improved over prior adenovectors by extending the 5' cis-acting packaging region further into the E1 gene to incorporate elements found to be important in optimizing viral packaging, resulting in enhanced virus amplification. Advantageously, these enhanced adenoviral vectors are capable of maintaining genetic stability following high passage propagation.

Standard techniques of molecular biology for preparing and purifying DNA constructs enable the preparation of the adenoviruses, shuttle plasmids, and DNA immunogens mentioned herein.

The vectors described above may be used in immunogenic compositions and vaccines for preventing or decreasing the likelihood of the development of adenocarcinomas associated with aberrant CEA, hTERT or her-2/neu expression and/or for treating existing cancers. The vectors allow for vaccine development and commercialization by providing an immunogenic CEA, hTERT or her-2/neu peptide which can elicit an enhanced immune response, relative to full-length wild-type CEA, hTERT or her-2/neu when administered to a mammal such as a human being.

In accordance with the method described above, the vaccine vector may be administered for the treatment or prevention of a cancer in any mammal, including but not limited to: lung cancer, breast cancer, and colorectal cancer. In a preferred embodiment of the invention, the mammal is a human. The cancer may be of the ovary, uterus, stomach, oesophagus, prostate or kidney.

Further, one of skill in the art may choose any type of vector for use in the treatment and prevention method described. Preferably, the vector is an adenovirus vector or a plasmid vector. In a preferred embodiment of the invention, the vector is an adenoviral vector comprising an adenoviral genome with a deletion in the adenovirus E1 region, and an insert in the adenovirus E1 region, wherein the insert comprises an expression cassette comprising: a polynucleotide comprising a sequence of nucleotides that encodes at least one immunogenic CEA, hTERT or her-2/neu analog as described herein and as set forth in SEQ ID NOs:3-7 and 9-13, and a promoter operably linked to the polynucleotide. In a preferred embodiment the adenovirus vector is an Ad 5 vector, an Ad6 vector, or an Ad 24 vector.

Also disclosed is a vaccine plasmid comprising a plasmid portion and an expression cassette portion, the expression cassette portion comprising: (a) a sequence of nucleotides that encodes an immunogenic T-cell peptide epitope analog of CEA as set forth in SEQ ID NOs:3-7 and 9-13; and (b) a promoter operably linked to the polynucleotide.

The amount of expressible DNA or transcribed RNA to be introduced into a vaccine recipient will depend partially on the strength of the promoters used and on the immunogenicity of the expressed gene product. In general, an immunologically or prophylactically effective dose of about 1 ng to 100 mg, and preferably about 0.25-5mg of a plasmid vaccine vector is administered directly into muscle tissue. An effective dose for recombinant adenovirus is approximately 10⁶ - 10¹² particles and preferably about 10⁷-10¹¹ particles.

It may be desirable for the vaccine vectors to be in a physiologically acceptable solution, such as, but not limited to, sterile saline or sterile buffered saline. Alternatively, it may be advantageous to administer an agent which assists in the cellular uptake of DNA, such as, but not limited to calcium ion. These agents are generally referred to as transfection facilitating reagents and pharmaceutically acceptable carriers. Those of skill in the art will be able to determine the particular reagent or pharmaceutically acceptable carrier as well as the appropriate time and mode of administration.

It is a common goal of vaccine development to augment the immune response to the desired antigen to induce long lasting protective and therapeutic immunity. Co-administration of vaccines with compounds that can enhance the immune response against the antigen of interest, known as adjuvants, has been extensively studied. In addition to increasing the immune response against the antigen of interest, some adjuvants may be used to decrease the amount of antigen necessary to provoke the desired immune response or decrease the number of injections needed in a clinical regimen to induce a durable immune response and to provide protection from disease and/or induce regression of disease.

Therefore, the vaccines and immunogenic compositions described herein may be formulated with an adjuvant in order to primarily increase the immune response. Adjuvants which may be used include, but are not limited to, adjuvants containing CpG oligonucleotides, (see A.M. Krieg, Biochimica et Biophysica Acta, 1489, 107-116, 1999) or other molecules acting on toll-like receptors such as TLR9 (for review, *see,* Daubenberger, C.A., Curr. Opin. Mol. Ther. 9(1):45-52 (2007)), T-helper epitopes, lipid-A and derivatives or variants thereof, liposomes, cytokines, (e.g. granulocyte macrophage-colony stimulating factor (GMCSF)), CD40, CD28, CD70, IL-2, heat-shock protein (HSP) 90, CD134 (OX40), CD137, non ionic block copolymers, incomplete Freund's adjuvant, and chemokines. Additional adjuvants for use with the compositions described herein are adjuvants containing saponins (e.g. QS21), either alone or combined with cholesterol and phospholipid in the characteristic form of an ISCOM ("immune stimulating complex," *for review, see* Barr and Mitchell, Immunology and Cell Biology 74: 8-25 (1996); and Skene and Sutton, Methods 40: 53-59 (2006)). Additionally, aluminum-based compounds, such as aluminum hydroxide (Al(OH)₃), aluminum hydroxyphosphate (AlPO₄), amorphous aluminum hydroxyphosphate sulfate (AAHS) or so-called "alum" (KAl(SO₄)·12H₂O), many of which have been approved for administration into humans by regulatory agencies worldwide, may be combined with the compositions provided herein.

As stated above, the epitopes and analogs and minigenes of the present invention can be included in an immunogenic composition or vaccine, which can then be administered to a human patient in need thereof to induce an immune response. Moreover, the administration of immunogenic compositions and vaccines comprising analogs to a patient in need thereof can effectively elicit an enhanced cellular immune response that is cross-reactive to human CEA, hTERT or her-2/neu protein relative to native epitopes.

Immunogenic compositions may be used alone at appropriate dosages which allow for optimal induction of a cellular immune response against CEA, hTERT or her-2/neu with minimal potential toxicity. In addition, co-administration or sequential administration of other agents may be desirable.

The compositions may be administered to a patient by intramuscular injection, subcutaneous injection, intradermal introduction, or impression though the skin. Other modes of administration such as intraperitoneal, intravenous, or inhalation delivery are also contemplated. In embodiments of the disclosure, the vaccines and pharmaceutical compositions are administered by intramuscular administration.

The minigenes of the present invention may be administered with an adjuvant, such as those described above. In particular, the adjuvant may be a natural or synthetic immunomodulatory oligodeoxynucleotide generally containing a CpG dinucleotide, particularly when this dinucleotide occurs in certain base contexts (CpG-S motifs). Alternatively the adjuvant may be an anti-CTLA4 antibody such as that described in Hodi et al, PNAS, 100, 4712-4717, 2003.

The minigenes of the present invention may also be administered simultaneously, sequentially or subsequently with a peptide containing promiscuous T-cell epitopes, such as tetanus toxoid peptide p2 or p30, see Valmori et al, *supra.*

Electroporation may be used to improve uptake of the minigenes according to the present invention into the subject to be vaccinated, for example using the method described in Babiuk et al, Vaccine, 20, 3399-3408, 2002.

The present invention thus also relates to a pharmaceutical composition comprising a minigene of the invention and a pharmaceutically acceptable excipient. The composition may be for the treatment or prophylaxis of cancer or an autoimmune disease or condition.

The present invention also relates to minigenes of the present invention for use in a method of treatment of the human body by therapy, such as the treatment or prophylaxis of cancer or an autoimmune disease or condition.

Thus, there is also disclosed a method of treating a subject suffering from or prone to cancer or an autoimmune disease or condition by therapy or prophylaxis which comprises administering to that subject a therapeutically or prophylactically effective amount of a minigene according to the present invention.

Disclosed is the use of a minigene of the invention for the manufacture of a medicament for the treatment or prophylaxis of cancer or an autoimmune disease or condition.

In general the minigene is administered to humans in an amount of 0.1-10 mg, particularly 1 to 5 mg, especially about 2.5 mg. The administration is generally intramuscular and is preferably followed by electroporation.

The following examples illustrate, but do not limit the invention.

### EXAMPLE 1

### Epitope Identification by EI Suite.

In order to identify epitope candidates that may elicit maximum tumor-specific immune response in a tolerized setting, while minimizing potential for off-target autoimmune activity, the entire CEA protein was scanned with several independent filters, using a proprietary software package called *EI Suite* (as described in WO 2006/124408). *EI Suite* ranks protein fragments based on binding affinity for a Class I MHC allele (in this case, HLA-A*0201), similarity to fragments of other human and murine proteins, and amenability to immunogenic enhancement.

A total of 12 HLA-A*0201 -restricted peptides and peptide analogs were identified by *EI Suite* as having the highest potential for use in an epitope-based vaccine or for *in vitro* monitoring of vaccine-induced CEA-specific CTL responses. The selected CEA peptide epitopes are shown in Table 1

| **Position** | **Peptide** | **SEQ ID NO.** | **Type** |
|---|---|---|---|
| CEA.691 | IMIGVLVGV | 1 | Wild type |
| CEA.411V10 | VLYGPDDPT**V** | 2 | Anchor-modified analog |
| CEA.690L2 | G**L**MIGVLVGV | 3 | Anchor-modified analog |
| CEA.589V10 | VLYGPDTPI**V** | 4 | Anchor-modified analog |
| CEA.682V10 | GLSAGATVG**V** | 5 | Anchor-modified analog |
| CEA.307V10 | GLNRTTVTT**V** | 6 | Anchor-modified analog |
| CEA.687 | ATVGIMIGV | 7 | Wild type |
| CEA.100V10 | IIYPNASLL**V** | 8 | Anchor-modified analog |
| CEA310L2 | R**L**TVTTITV | 9 | Anchor-modified analog |
| CEA605V9 | YLSGANLN**V** | 10 | Anchor-modified analog |
| CEA687L2 | A**L**VGIMIGV | 11 | Anchor-modified analog |
| CEA691L2 | I**L**IGVLVGV | 12 | Anchor-modified analog |

and include 2 wild-type CEA peptides and 10 peptide analogs. Examples 2-4 provide the details of peptide selection.

### EXAMPLE 2

The A*0201 binding affinity filter.

HLA binding affinity has been shown to correlate with T-cell recognition for peptides derived from viral antigens (Sette et al. J. Immunol. 153: 5586-92 (1994)). More recently, this relationship has also been observed for tumor epitopes (Keogh et al. J. Immunol. 167: 787-796 (2001)). Selecting potential epitopes on the basis of HLA binding affinity is, therefore, an efficient alternative to large-scale epitope mapping or other T cell-dependent strategies.

Therefore, using *EI Suite,* the sequence of the CEA protein was scanned for peptides that were predicted to be strong binders to the HLA class I allele A*0201 (referred to as A2.1 below). A total of 1267 peptides representing all possible 9- and 10-mer frames of the 702 amino acid protein were evaluated. Each peptide was scored based on the degree of adherence to a statistical motif inferred from several publicly available epitope databases, including: SYFPEITHI (Rammensee et al. Immunogenetics 50: 213-19 (1999)), MHCBN (Bhasin et al. Bioinformatics 19(5): 665-66 (2003), and FIMM (Schönbach et al. Nucleic Acids Res. 28(1): 222-24 (2000)). 150 top-scoring fragments were retained for further analysis.

### EXAMPLE 3

### Comparison of CEA peptides with fragments of other human proteins.

A vaccine containing only CEA-specific epitopes may prevent the off-target T-cell response that sometimes occurs when immune tolerance is broken to a shared antigen. The CTL-mediated destruction of melanocytes (van Elsas et al. J. Exp. Med. 190: 355-366(1999)) and pancreatic islet β-cells (Ludewig et al. J. Exp. Med. 2000, 191: 795-804 (2000)) following immunotherapy are two examples of such off-target immune response. Moreover, CEA peptides that are also found in other proteins that are expressed in multiple normal tissues are more likely to have been presented to T-cells in a tolerizing setting. Immune tolerance to such peptides may therefore be more difficult to overcome. For this reason, *EI Suite* was used to select, from the 150 top-scoring CEA peptides, those unique to the CEA protein.

It is known that T-cells can often recognize cognate epitopes comprising modifications at the HLA contact positions (positions 2 and 9/10 for HLA-A2.1) (Keogh (2001), *supra*)*.* In contrast, mismatches within the TCR contact region (positions 1 and 3-8/9, for HLA-A2.1) are generally expected to lead to a loss of recognition. Therefore, to minimize the likelihood of vaccine-induced autoimmunity, CEA peptides whose TCR contact region was identical to a fragment of another human protein were rejected as epitope candidates even if they were predicted to be strong HLA binders and potentially immunogenic. Of 150 top-scoring CEA peptides, 49 were rejected because their TCR contact residues were identical to a fragment of another human protein.

However, a certain degree of degeneracy in peptide recognition exists with respect to changes in the TCR contact region (Sparbier et al. Curr. Opin. Imm. 11: 214-21 (1999)). In fact, depending on the peptide and the T-cell clone, changes in the TCR contact region may result in an enhanced recognition, loss of recognition, or an intermediate response (Scardino et al. Eur. J. Imm. 31: 3261-3270 (2001)). In light of this fact, an expanded search of the human proteome was performed, allowing a single mismatch in the TCR binding region, in addition to a mismatch, if any, at positions 2 or 9/10. Of 150 top-scoring CEA peptides, 105 had sufficiently close matches with other proteins by this definition. These peptides were flagged to indicate that, if selected as vaccine candidates, they must undergo additional screening to rule out cross-recognition of proteins other than the target protein CEA. Interestingly, 15/150 (10%) top-scoring CEA peptides had matches outside the CEA family.

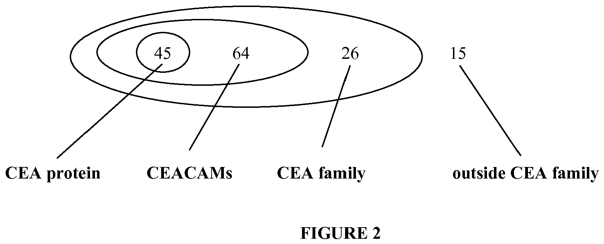

The output of the self-similarity filter for the 150 top-scoring CEA peptides is summarized in FIGURE 2. Most of the matches were within proteins in the CEACAM branch (CEA-like cell adhesion molecule) of the CEA family (Nomenclature Announcement, Exp. Cell Res. 252: 243-249 (1999)). In fact, 44/150 (29%) top-scoring CEA peptides were identical to a fragment of another CEACAM, and a few occurred in a majority of expressed human CEACAMs, including biliary glycoprotein (BGP, CEACAM1) and non-specific cross-reacting antigen (NCA, CEACAM6). BGP and NCA are both known to be expressed broadly in normal epithelia, including pancreas, lung, liver, kidney, and cervix (Hammarstrom et al. Semin Cancer Biol. 9: 67-81 (1999)). NCA is also known to be overexpressed in colorectal carcinoma (Koops et al. Eur J Biochem 253(3): 778-786(1998)).

17% (26/150) top-scoring CEA peptides matched a pregnancy-specific glycoprotein (PSG) (Hammarstrom *et al.* 1999, *supra*)*.* CEA-like cell adhesion molecules (CEACAM) and pregnancy-specific glycoproteins (PSG) form two branches of the CEA family, with CEA being in the CEACAM subset. PSGs have a unique expression profile compared to CEACAM members, with ubiquitous expression noted in the placenta during fetal development, and a more restrictive expression pattern seen in the normal adult tissues pancreas, salivary glands, and brain (Hammarstrom et al. 1999, *supra*)*.*

### EXAMPLE 4

### Anchor-modified peptide analogs.

It is known that the use of peptides modified at one or more HLA contact positions, called anchor-modified peptide analogs, may improve HLA binding affinity (*See, e.g.* Lipford et al. Immunology 84: 298-303 (1998)). As a result of improved HLA binding, the modified peptide is often more immunogenic than the original wild-type (wt) peptide. Furthermore, analog-specific T-cells generally recognize the wt peptide because of the identical amino acid residues in the TCR contact region.

Therefore, anchor-modified immunogenic analogs were identified for 11 of 14 CEA peptides that were not rejected by the self-similarity filter and were predicted to be moderate or better A2.1 binders (the score of 2 or higher). Most of the analogs identified comprise an I to V mutation at the C-terminus. Peptides with this mutation had a greater affinity for A2.1 and elicited T-cells that recognized the wt peptide more consistently than any other alteration in the HLA contact region (Keogh et al. J. Immunol. 167: 787-796 (2001)). Analog CEA.690L2 was not modified at the C-terminus because the wt peptide CEA.690 already comprises a valine in this position. Instead, a substitution to a leucine, a preferred amino acid at position 2 in the A*0201 binding motif, was made to improve binding affinity.

Anchor modifications for weaker HLA binders (binding score <2) were not investigated. These peptides are thought to be present at low density on the cell surface in complex with the HLA molecule, having been out-competed by stronger binding peptides (Pardoll, D.M. Nat Rev Imm 2: 227-238 (2002)).

### EXAMPLE 5

### Binding affinity of T-cell Epitopes to T2 cells.

To determine the binding ability of *EI* Suite-selected wild type peptides and analogs, an *in vitro* cellular binding assay was performed using the TAP-deficient cell line T2. T2 cells are HLA-A*0201 positive, MHC class II negative and TAP deficient, meaning that they lack a functional transporter associated with antigen presentation, so that they accumulate empty unstable class I molecules.

T2 cells were incubated with 50 µM peptide in serum-free RPMI 1640 supplemented with 5 µg/mL human β2m (Fluka) for 18 hours at 37°C. HLA-A*0201 expression was then measured by flow cytometry using the anti-HLA-A2.1 monoclonal antibody (mAb) BB7.2 followed by incubation with fluorescein isothiocyanate (FITC)-conjugated F(ab')2 goat antimouse Ig (Biosource). The results are expressed as fluorescence index (FI) defined as a ratio (median channel of fluorescence) between the sample and a control without any peptide. An increase over the control of at least 65% (FI > 2) was arbitrarily chosen as the cutoff point.

The binding assay consisted of the exogenous addition of peptides and β2 microglobulin protein: peptide binding up-regulates surface HLA expression and HLA-A2 molecules on the surface are measured using FACS by means of an antibody capable of recognizing the peptide-MHC complex (Kuzushima et al. Blood; 98:1872-81 (2001); Passoni et al. Blood 99:2100-06 (2002)).

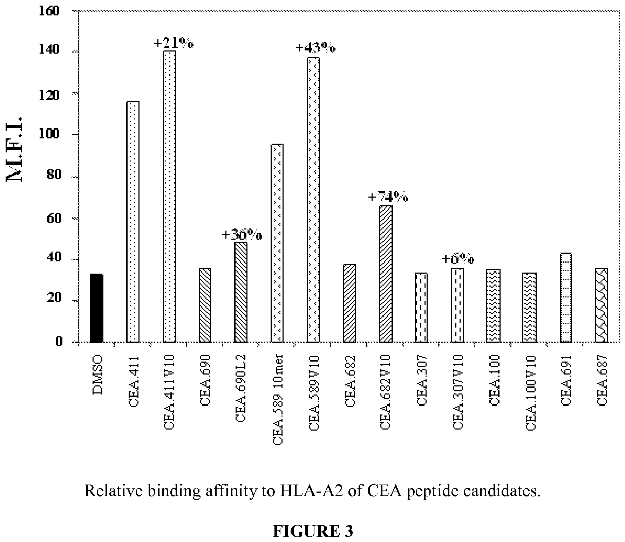

Results of the binding assay are shown in FIGURE 3. Most of the modified epitopes (411V10, 690L2, 589V10, 682V10, 307V10) showed a marked increase of binding to HLA-A*0201 compared to their wild-type counterparts.

### EXAMPLE 6

### Measurement of HLA-A*0201/peptide complex stability

To assess the HLA-A*0201/peptide complex stability, T2 cells (10⁶/mL) were incubated overnight with 50 µM of each peptide in serum-free RPMI 1640 supplemented with 100 ng/mL human β2m at 37°C. Cells were then washed 4 times to remove free peptides, incubated for 1 hour with 10 µg/mL Brefeldin A (Sigma-Aldrich) to block cell surface expression of newly synthesized HLA-A*0201 molecules, washed, and incubated at 37°C for 0, 2, 4, 6, or 8 hours. Subsequently, cells were stained with anti-HLA-A2.1 mAb BB7.2. For each time point, peptide-induced HLA-A*0201 expression was calculated as mean fluorescence value of peptide incubated T2 cells/mean fluorescence value T2 cells in the absence of the peptide. Dissociation complex 50 (DC50) was defined as the time required for the loss of 50% of the HLA-A*0201/peptide complexes stabilized at t = 0.

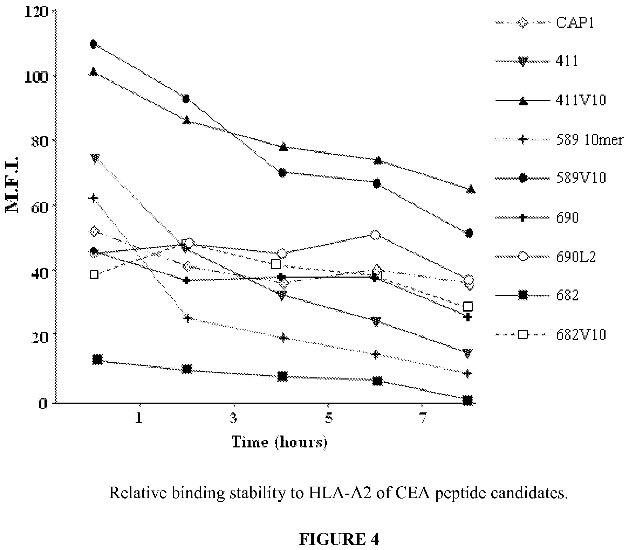

Binding stability of the peptide-MHC complex was also evaluated over time. The CAP-1 peptide was used as positive control. Again, results demonstrate an improved stability of peptide analogs, in particular for 411V10, 589V10 and 682V10, compared to their wild-type counterparts (FIGURE 4).

### EXAMPLE 7

### Immunogenicity of T-cell epitopes in HHD transgenic mice.

HLA-A2.1 (HHD) transgenic mice were bred at Charles River Laboratories (Lecco, Italy). These mice are transgenic for the HHD complex (human β2-microglobulin fused to HLA-A2.1 α1 and α2 domain, H-2D^{b} α3 domain) and are devoid of H-2D^{b} and murine β2-microglobulin. (Pascolo et al. J. Exp. Med. 185(12):2043-51 (1997)). For this reason, the immune response elicited in these mice is specifically restricted to human HLA-A2.1, making this line a suitable model for epitope identification and optimization.

To determine the *in vivo* immunogenicity of CEA analogs and wild-type peptides, HHD transgenic mice were co-immunized with 100 µg of the CEA peptide and 140 µg of the HBV core 128 helper T cell peptide (I-A^{b}-restricted, sequence TPPAYRPPNAPIL (SEQ ID NO:16). Both immunogens were emulsified in incomplete Freund's adjuvant (IFA) together with 50 µg of CpG TLR9 agonist and the emulsion was injected subcutaneously (s.c.) at the tail base of each animal.

Groups of 4 HHD mice were injected subcutaneously with the following components: 1) wild type peptide or analog (100 mcg); 2) HBV core peptide (140mcg); 3) CpG oligonucleotide (50mcg); 4) incomplete Freund adjuvant. Mice were given a second injection 15 days later with the same component.

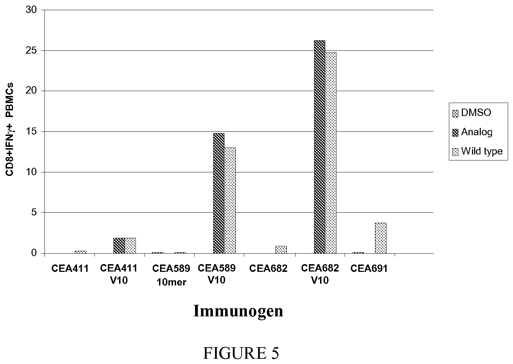

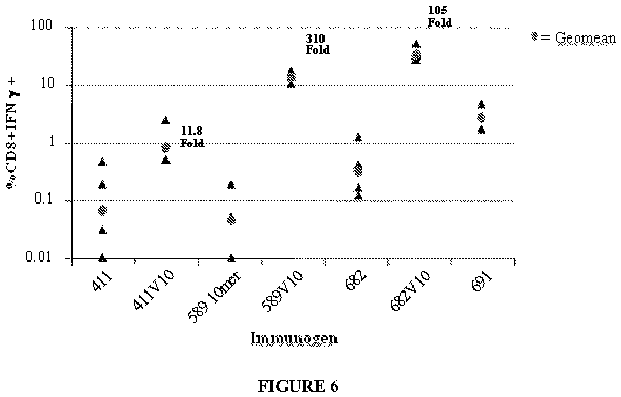

Two weeks after the last injection, mice were bled and peripheral blood lympho-monocytes (PBMC) and/or splenocytes were recovered for immunological assays. PBMC or splenocytes were analyzed by intracellular staining (ICS) for interferon gamma release upon stimulation with wild type or analog peptides (see EXAMPLE 8). Results demonstrate that the peptide analogs were more immunogenic than their wild type counterparts (FIGURES 5 and 6). The enhancement in immune response for the peptide analogs relative to the corresponding wt peptide ranged from 11.8- to 310-fold depending on the epitope. Importantly, the response elicited by analogs was fully cross reactive with corresponding natural peptides.

### EXAMPLE 8

### Intracellular Staining (ICS) for IPNγ.

One to two millions mouse splenocytes or PBMC in 0.6ml RPMI 10% FCS were incubated with peptides (5 µg/ml final concentration of each peptide) and brefeldin A (1 µg/ml; BD PharMingen, Franklin Lakes, NJ) at 37°C and 5% CO₂ for 12-16 hours. Cells were then washed with FACS buffer (PBS 1% FBS, 0.01% NaN₃) and incubated with purified anti-mouse CD16/CD32 Fc block (BD PharMingen) for 15 min at 4°C. Cells were then washed and stained with surface antibodies: CD4-PE conjugated anti-mouse (BD PharMingen), PercP CD8 conjugated anti mouse (BD PharMingen) and APC- conjugated anti-mouse CD3e (BD PharMingen) for 30 minutes at room temperature in the dark. After the washing, cells were fixed and permeabilized with Cytofix-Cytoperm Solution (BD PharMingen) for 20 min at 4°C in the dark. After washing with PermWash Solution (BD PharMingen) cells were incubated with the IPNγ-FITC antibodies (BD PharMingen). Cells were then washed, fixed with formaldehyde 1% in PBS and analyzed on a FACS-Calibur flow cytometer, using CellQuest software (Becton Dickinson, Franklin Lakes, NJ).

### EXAMPLE 9

### Identification of additional immunogenic CEA epitopes

Additional HLA-A2.1 restricted peptides from CEA were identified by *EI Suite* as potential immunogenic epitopes for peptide and/or minigene vaccine, using the procedure described in EXAMPLES 1-3. These peptides were modified at specific positions as described in Example 4, to increase their binding affinity to MHC-I and consequently enhance their immunogenic potency.

In order to assess the immunogenicity of these peptides and correlate the immunogenic outcome with biochemical binding properties, groups of 6 HHD mice were immunized subcutaneously with 100µg of each peptide admixed with HBVcore128 helper epitope and a TLR9 agonist (Coley's CpG) in incomplete Freund adjuvant. Two weeks later, mice received a second injection with the same peptide mixture. After three weeks the immune response against the natural target epitope was analyzed by intracellular staining for IPNγ □□ as described in EXAMPLE 8. Results show that CEA691 and CEA605 were immunogenic, but little or no improvement was conferred by the corresponding analogs (FIGURE 7). On the other hand, CEA310 epitope was poorly immunogenic while CEA310L2 analog was extremely powerful in eliciting a cross reactive immune response. In fact, this analog was 123 fold more immunogenic than the natural peptide. Similarly, CEA687 was found to be significantly immunogenic in mice, resulting in 4 out of 6 mice responding to the vaccination. However, the analog CEA687L2 was about 3.4 fold more immunogenic and 100% of the mice responded to the treatment. Importantly, a very good correlation between *in vitro* binding affinity (i-Topia assay) and *in vivo* immunogenicity data was found for these epitopes (data not shown)..

These data demonstrate that CEA691L2, CEA605V9, CEA310L2 and CEA687L2 would be useful for the development of a peptide-based vaccine targeting CEA, or for the construction of an epitope modified minigene (EMM) vaccine, or any other modality that incorporates these analogs, such as a genetic vaccine encoding CEA, in which positions 691-699, 310-318, etc, are replaced with the corresponding analog sequences, as described throughout the specification.

### EXAMPLE 10

### T-cell Epitope List for hTERT

2247 peptides representing all possible 9- and 10-mer frames of hTERT protein (SwissProt accession number 014746) were ranked using *EI Suite* starting from the full hTERT sequence as described above for CEA, and a total of 12 peptides were selected, including eight wild type epitopes and four analogs (Table 2).

### Table 2: hTERT epitope candidates and analogs selected by EI Suite.

Modified amino acid residues are highlighted.

| **Position in hTERT** | **Length** | **Type** | **Sequence** | **Wild type sequence** | **SEQ ID NO:** |
|---|---|---|---|---|---|
| 30 | 9 | w.t. | RLGPQGWRL | | 13 |
| 540 | 9 | w.t. | ILAKFLHWL | | 14 |
| 540 | 9 | V9 | ILAKFLHWV | ILAKFLHWL | 15 |
| 540 | 10 | w.t. | ILAKFLHWLM | | 16 |
| 540 | 10 | V10 | ILAKFLHWLV | ILAKFLHWLM | 17 |
| 544 | 10 | w.t. | FLHWLMSVYV | | 18 |
| 688 | 10 | w.t. | RAWRTFVLRV | | 19 |
| 688 | 10 | L2 | RLWRTFVLRV | RAWRTFVLRV | 20 |
| 865 | 9 | w.t. | RLVDDFLLV | | 21 |
| 865 | 9 | F1 | **F**LVDDFLLV | RLVDDFLLV | 22 |
| 934 | 9 | w.t. | LLDTRTLEV | | 23 |
| 986 | 9 | w.t. | FLDLQVNSL | | 24 |

### EXAMPLE 11

### In-vitro binding of EI Suite-selected hTERT epitopes and analogs to HLA-A*0201

Candidate epitopes and analogs are analyzed for the ability to bind to HLA-A*0201 in-vitro using iTopia™ Epitope Discovery System (http://www.immunomics.com). This assay provides a method to determine the binding affinity and stability of a test peptide to any of several Class I HLA molecules, using fluorescent-labeled antibody that specifically recognizes HLA only when peptide is bound in the MHC-I groove.

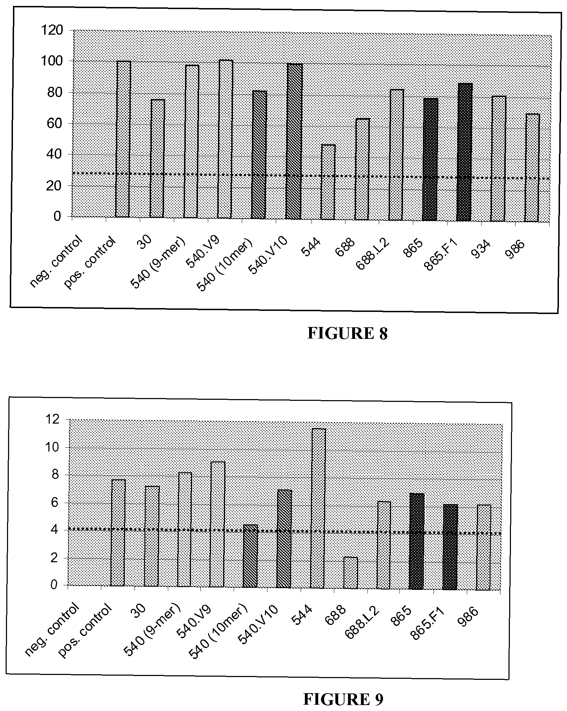

The binding affinity to HLA-A*0201 of EI Suite-selected epitopes and analogs are summarized in Fig. 8. Eight out of 8 wild type peptides show strong or medium binding to HLA-A*0201, as evidenced by a binding affinity of 30% of the positive control or greater (the 30 % threshold is suggested in the iTopia™ technical manual). In addition, four out of 4 analogs (540.V9, 540.V10, 688.L2, and 865.F1) show an increase in binding affinity to HLA-A*0201 compared to the corresponding wild type peptide.

Similarly, the stability of the peptide-HLA complex is evaluated over time (Fig. 9). The data shows that 10/11 peptides tested have an off-rate of 4 hr or greater (off-rate data is unavailable for peptide hTERT.934). Furthermore, three out of 4 analogs show an improved stability compared to the corresponding wild type peptide.

### EXAMPLE 12

### Immunogenicity of T-cell Epitopes in HHD mice.

HHD transgenic mice carry the HLA-A*0201/Db (or HHD) fusion protein and are knock-out for murine MHC class I molecule. For this reason, the immune response elicited in these mice is specifically restricted to the human HLA-A*0201 allele, making this model suitable for epitope identification and optimization.

Groups of five or six HHD mice are injected subcutaneously with the following components: 1) wild type peptide or analog (50 mcg); 2) HBV core peptide (140mcg); 3) CpG oligonucleotide (50mcg); 4) Incomplete Freund Adjuvant. A second injection is performed 15 days later.

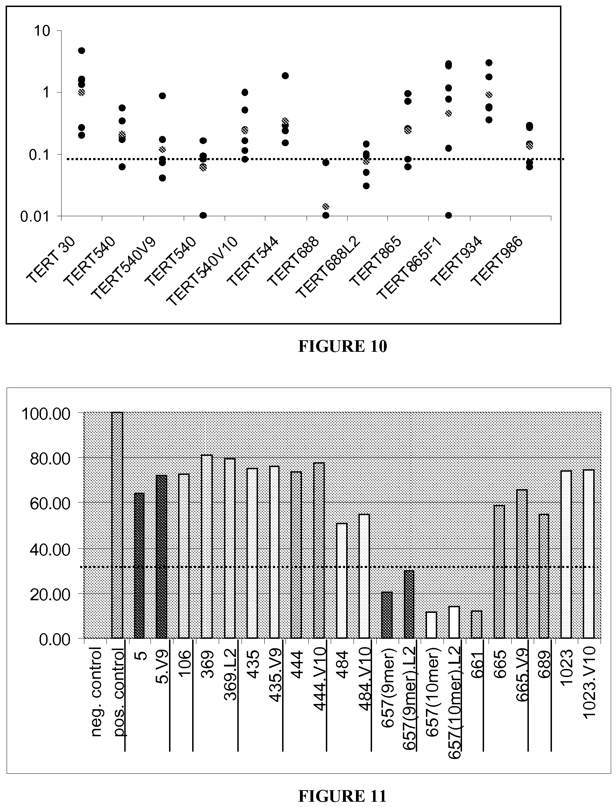

Two weeks after the last injection, mice are sacrificed, their splenocytes collected, and cell mediated immune response against wild type epitopes is measured for each mouse separately by ICS (Fig. 10).

Nine out of 12 EI Suite-suggested peptides elicit a significant cell-mediated response, defined as the mean response of 0.1% CD8+IFNg+ or greater. Furthermore, four out of 4 analogs show an enhanced immunogenicity against the wild type peptide, compared to when the wild type peptide is used as an immunogen.

### EXAMPLE 13

### List of T-cell Epitopes and Analogs for HER2

2493 peptides representing all possible 9- and 10-mer frames of HER2 protein (SwissProt accession number P04626) were ranked using *EI Suite* from the sequence of her-2/neu as described above for CEA, and a total of 21 peptides were selected, including twelve wild type epitopes and nine analogs (Table 3).

**Table 3: HER2 epitope candidates and analogs selected by EI Suite.**

| Modified amino acid residues are highlighted. | | | | | |
|---|---|---|---|---|---|
| Position in HER2 | Length | Type | Sequence | Wild Type Sequence | SEQ ID NO |
| 5 | 9 | w.t. | ALCRWGLLL | | 25 |
| 5 | 9 | V9 | ALCRWGLLV | ALCRWGLLL | 26 |
| 106 | 9 | w.t. | QLFEDNYAL | | 27 |
| 369 | 9 | w.t. | KIFGSLAFL | | 28 |
| 369 | 9 | L2 | KLFGSLAFL | KIFGSLAFL | 29 |
| 435 | 9 | w.t. | ILHNGAYSL | | 30 |
| 435 | 9 | V9 | ILHNGAYSV | ILHNGAYSL | 31 |
| 444 | 10 | w.t. | TLQGLGISWL | | 32 |
| 444 | 10 | V10 | TLQGLGISWV | TLQGLGISWL | 33 |
| 484 | 10 | w.t. | QLFRNPHQAL | | 34 |
| 484 | 10 | V10 | QLFRNPHQAV | QLFRNPHQAL | 35 |
| 657 | 9 | w.t. | AVVGILLVV | | 36 |
| 657 | 9 | L2 | ALVGILLVV | AVVGILLVV | 37 |
| 657 | 10 | w.t. | AVVGILLVVV | | 38 |
| 657 | 10 | L2 | ALVGILLVVV | AVVGILLVVV | 39 |
| 661 | 10 | w.t. | ILLVVLGVV | | 40 |
| 665 | 9 | w.t. | VVLGVVFGI | | 41 |
| 665 | 9 | V9 | VVLGVVFGV | VVLGVVFGI | 42 |
| 689 | 9 | w.t. | RLLQETELV | | 43 |
| 1023 | 10 | w.t. | YLVPQQGFFC | | 44 |
| 1023 | 10 | V10 | YLVPQQGFFV | YLVPQQGFFC | 45 |

### EXAMPLE 14

### In-vitro binding of EI Suite-selected HER2 epitopes and analogs to HLA-A*0201

Candidate epitopes and analogs are analyzed for the ability to bind to HLA-A*0201 in-vitro using iTopia™ Epitope Discovery System (http://www.immunomics.com). This assay provides a method to determine the binding affinity and stability of a test peptide to any of several Class I HLA molecules, using fluorescent-labeled antibody that specifically recognizes HLA only when peptide is bound in the MHC-I groove.

The binding affinity to HLA-A*0201 of EI Suite-selected epitopes and analogs are summarized in Fig. 11. Nine out of twelve wild type peptides show strong or medium binding to HLA-A*0201, as evidenced by a binding affinity of 30% of the positive control or greater (the 30% threshold is suggested in the iTopia™ technical manual). In addition, eight out of nine analogs show an increase in binding affinity to HLA-A*0201 compared to the corresponding wild type peptide.

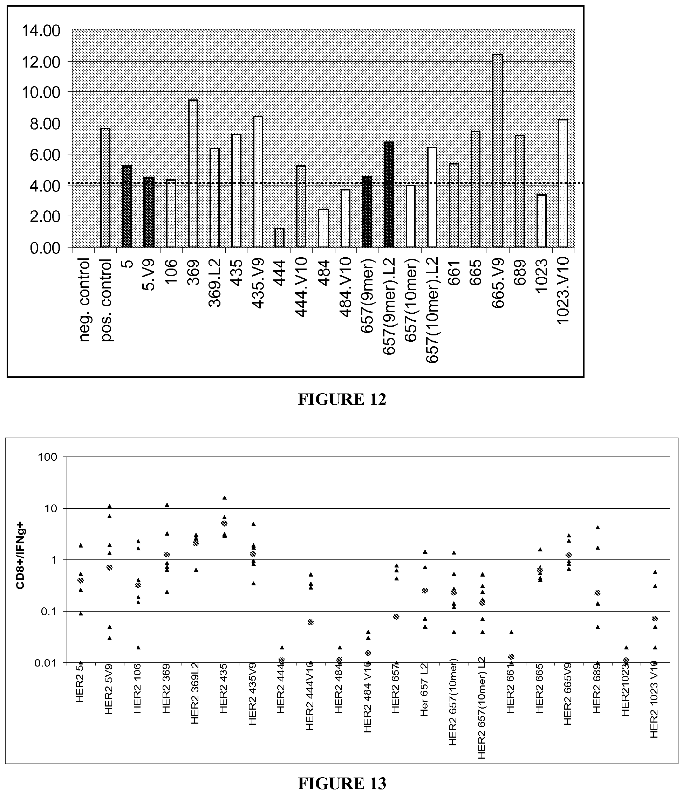

Similarly, the stability of the peptide-HLA complex is evaluated over time (Fig. 12). The data shows that 17/21 peptides tested have an off-rate of 4 hr or greater. Furthermore, seven out of 9 analogs show an improved stability compared to the corresponding wild type peptide.

### EXAMPLE 15

### Immunogenicity of T-cell Epitopes in HHD mice.

HHD transgenic mice carry the HLA-A*0201/Db (or HHD) fusion protein and are knock-out for murine MHC class I molecule. For this reason, the immune response elicited in these mice is specifically restricted to the human HLA-A*0201 allele, making this model suitable for epitope identification and optimization.

Groups consisting of 4 HHD mice are injected subcutaneously with the following components: 1) wild type peptide or analogs (100mcg); 2) HBV core peptide (140mcg); 3) CpG oligonucleotide (50mcg); 4) Incomplete Freund Adjuvant. A second injection was performed 14 days later.

Two weeks after the last injection mice are sacrificed, their splenocytes are collected, and cell mediated immune response against wild type epitopes is measured for each single mouse by ICS. Thirteen out of twenty one peptides elicit a significant cell-mediated response, defined as the mean response of 0.1 %CD8+IFNg+ or greater (Fig. 13). Furthermore, seven out of nine analogs show an enhanced immunogenicity against the wild type peptide, compared to when the wild type peptide is used as an immunogen.

### EXAMPLE 16

### Example of a minigene scaffold.

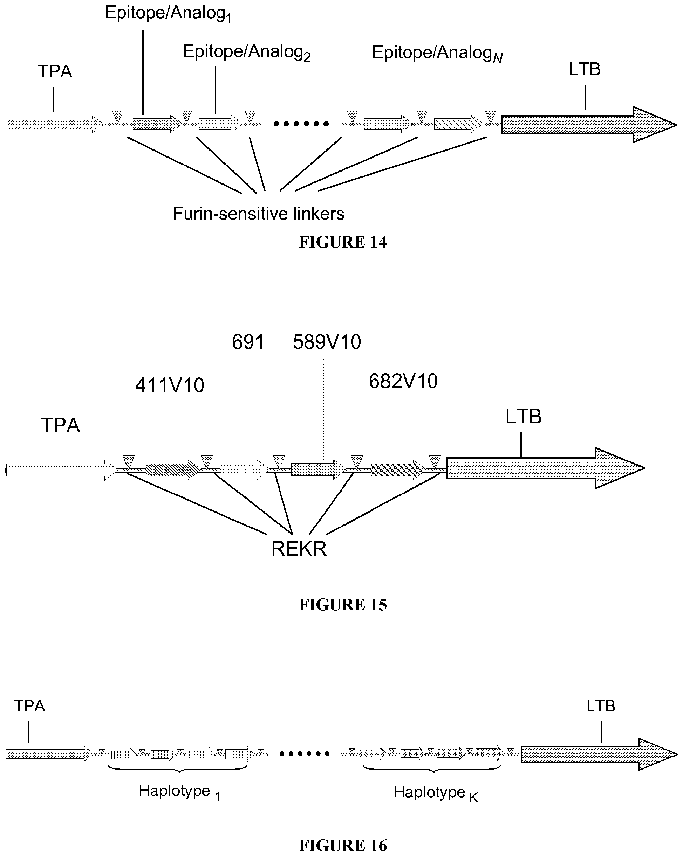

A minigene exemplifying the scaffold defined in this invention is shown in Figure 15. The minigene contains four epitopes and immunogenic analogs from the carcinoembryonic antigen (CEA) previously shown to be immunogenic individually in HHD/CEA double transgenic mice and to elicit responses cross-reactive with the wild-type epitope. The epitopes and analogs are as follows:
1. CEA.411V10 (VLYGPDDPTV) (SEQ ID NO:2);
2. CEA.691 (IMIGVLVGV) (SEQ ID NO:12),
3. CEA.589V10 (VLYGPDTPIV) (SEQ ID NO:4), and
4. 682V10 (GLSAGATVGV) (SEQ ID NO:5).

The epitopes/analogs are linked by furin-sensitive linker REKR.

### EXAMPLE 17

### The modular structure of minigenes containing epitopes from multiple antigens and restricted by multiple HLA alleles.

Any one or more confirmed or predicted epitopes or analogs from any tumor-associated or microbial antigen, or multiple antigens, could be used in place of, or in addition to, the four sequences used in Example 16. For example, one or more of the HLA-A*0201-restricted epitopes and analogs identified from CEA, her-2/neu or hTERT identified above. Predicted or confirmed epitopes or analogs restricted by MHC alleles other than HLA-A*0201, or epitopes restricted by a combination of MHC alleles, may also be used (see Figure 16). Furthermore, some or all of the epitopes/analogs may be repeated two or more times. Finally, any other furin-sensitive linker, or a combination of furin-sensitive linkers, may be used.

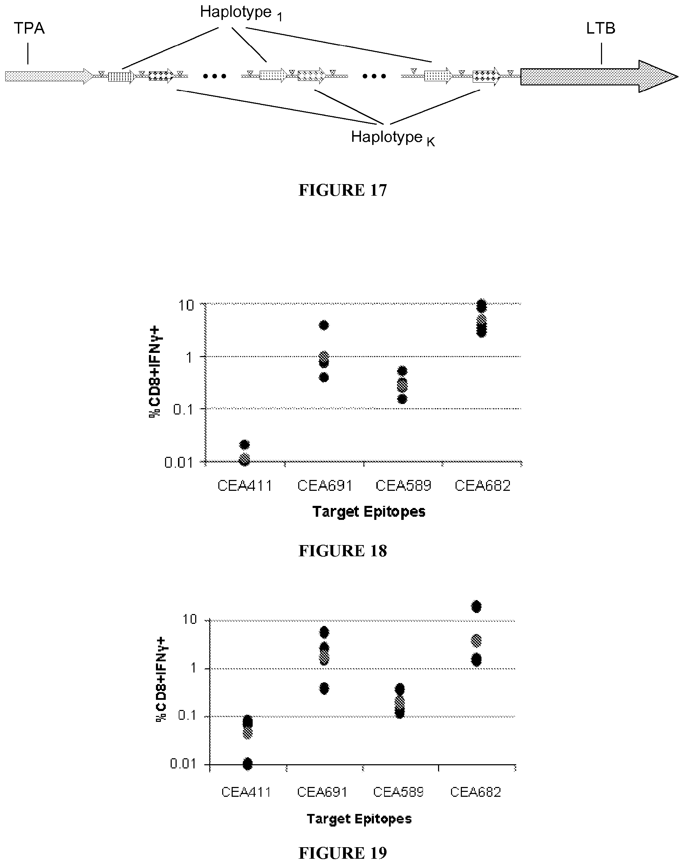

An alternate design of such a minigene can also be considered where neighboring epitopes (from one or more antigens) are restricted by different MHC alleles (Figure 17).

### EXAMPLE 18

### The minigene of Example 16 generates significant immune response in-vivo against individual epitopes.

HHD/CEA double transgenic mice were generated by crossing HHD (Pascolo et al. J. Exp. Med 1997; 185: 2043-2051) with CEA transgenic mice (Clarke et al., Cancer Res 1998;58:1469-779). This mouse model allows to assess and measure the immunogenicity of a CEA cancer vaccine in the presence of immunologic tolerance, thus providing an useful and more predictive tool. These mice were used to evaluate the immunogenicity of the scaffold shown in Figure 15. Five HHD/CEA mice were injected intramuscularly with 50µg of the minigene followed by electroporation (EP). On days 7, 14, and 21, mice were boosted with additional injections. On day 40, mice were sacrificed and splenocytes were stimulated with the wild type epitopes. Results are shown in Figure 18. It is evident that vaccination with this minigene generated significant immune responses against CEA691 and the wild-type epitopes corresponding to two of the three analogs (589V 10, and 682V10) included in the minigene. However, 411 epitope show poor reactivity. The possible reasons for this observation are: 1) low immunogenicity of 411V10 analog; 2) position-dependent lack of epitope processing within the folded polypeptide; 3) epitope competition within the same antigen-presenting cell (APC).

### EXAMPLE 19

### Further evidence that the minigene of Example 16 generates significant immune response in-vivo.

The minigene of Example 16 was tested in an independent experiment in four additional HHD/CEA mice. The results, shown in Figure 19, confirm that the scaffold of Example 16 reproducibly elicits robust immune responses against the epitopes in the minigene.

### EXAMPLE 20

### Addition of a helper epitope (p30) to the minigene scaffold

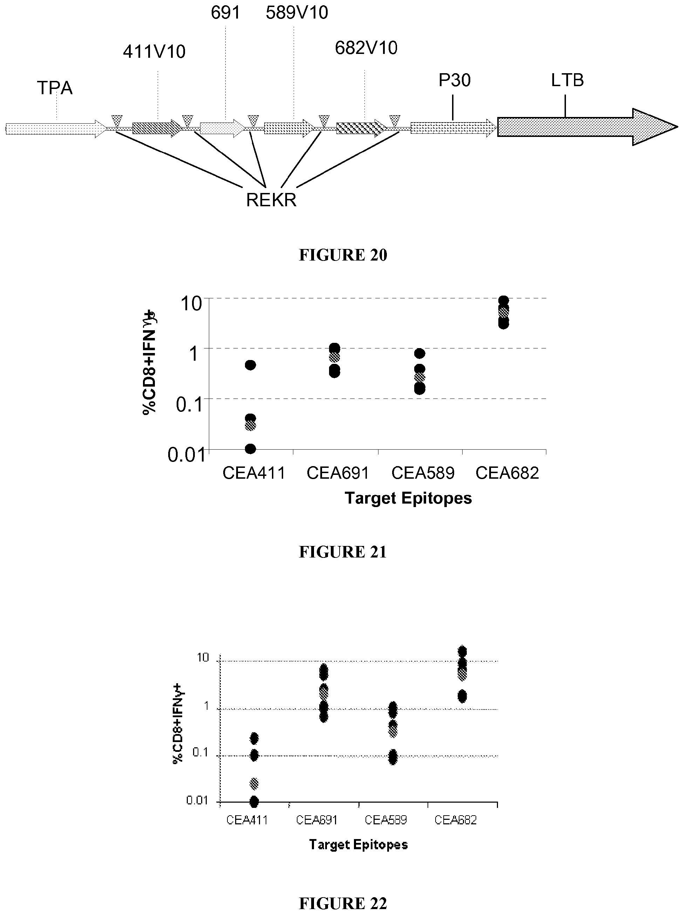

The modular structure of the minigene scaffold described in this invention allows for the inclusion of a separate source of T-helper epitopes, which have been shown to increase CD8⁺ cell mediated immune response. For example, minigene shown in Figure 20 contains a tetanus toxin-derived universal helper peptide p30.

### EXAMPLE 21

### A separate helper epitope is not necessary for the construct in Example 16.

To evaluate whether addition of p30 could further increase the immunogenicity of the construct in Example 16, five HHD/CEA mice were immunized with the p30-containing minigene described in Example 20. The immunization protocol was the same as described in Example 3. The immune response against the wild type peptides, shown in Figure 21, was of similar magnitude compared to that elicited by the minigene of Example 16, which did not contain p30 (compare Figure 21 with Figure 18 in Example 18).

The addition of a universal helper epitope does not improve the immunogenicity of the scaffold in Example 16.

### EXAMPLE 22

### Further evidence that a separate helper epitope is not necessary for the construct of Example 16.

Four additional HHD/CEA mice were immunized with the p30-containing minigene of Example 20 using the protocol of Example 19. The immune response, shown in Figure 22, was again similar to that generated by the construct of Example 16 which did not contain p30 (compare Figure 22 with Figure 19 in Example 19).

### EXAMPLE 23

### The immune response is independent of the epitope position in the minigene.

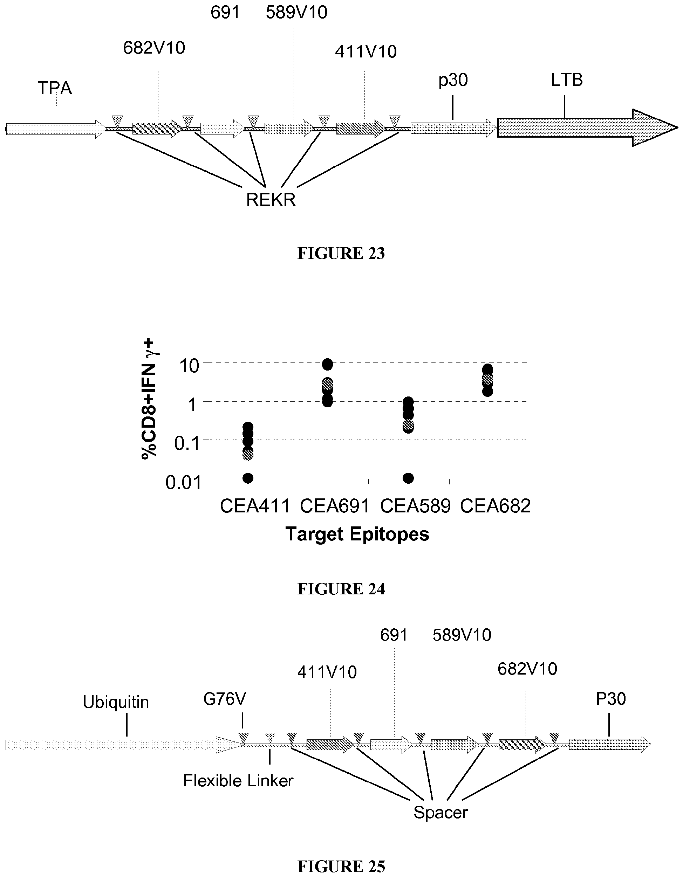

The protocol of Example 19 was used to test if the immune response against the wild-type epitopes is dependent on the position in the minigene where each epitope or analog appears. Consider a minigene construct similar to that of Example 22, except the analogs 411V10 and 682V10 were switched (see Figure 23).

The results, shown in Figure 24, exhibit no significant differences between immune responses against all four wild-type epitopes compared to those in Example 22.

### EXAMPLE 24

### The immune response generated by the minigene of Example 16 is higher than that elicited by proteasome-targeting minigenes

In a variant construct (see Figure 25), the minigene comprised of the same four epitope/analogs was fused to a mutant form of ubiquitin (G76V) (see Stack et al, Nature Biotech., 18, 1298-1302, 2000) via a flexible linker peptide (VGKGGSGG (SEQ ID NO:48)). Ubiquitin fusion has been shown to improve the induction of CD8⁺ T-cell response by targeting the protein for rapid degradation by the proteasome (see Rodriguez et al, J. Virol, 72, 5174-5181, 1998). The design also included the use of one of three spacer sequences, AAY (see Wang et al, Sc. J. Immunol., 60, 219-225, 2004), LRA, or RLRA, designed to ensure efficient epitope processing by the proteasome.

HHD/CEA mice (n=5 per group) were immunized with a minigene shown in Figure 25 containing either AAY or LRA as spacers, using the immunization protocol of Example 17. A similar test was conducted for the minigene scaffold using the spacer RLRA and the protocol of Example 18.

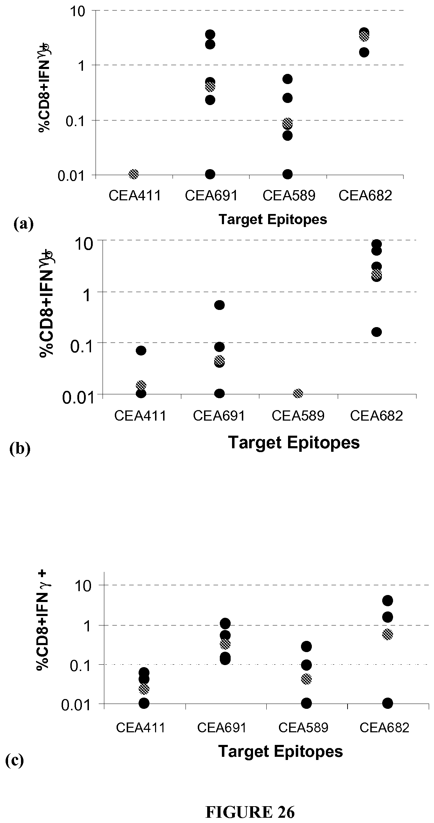

The results, as shown in Figure 26, lead to the conclusion that the minigene of Example 16 performs at least as well as any of the three ubiquitin-containing constructs, if not better (compare results in Figure 26a-b with that of Figure 18, and the results in Figure 26c with Figure 19).

### EXAMPLE 25

### The immune response generated by the minigene of Example 1 is not improved by replacing LTB with a membrane-translocating sequence (MTS).

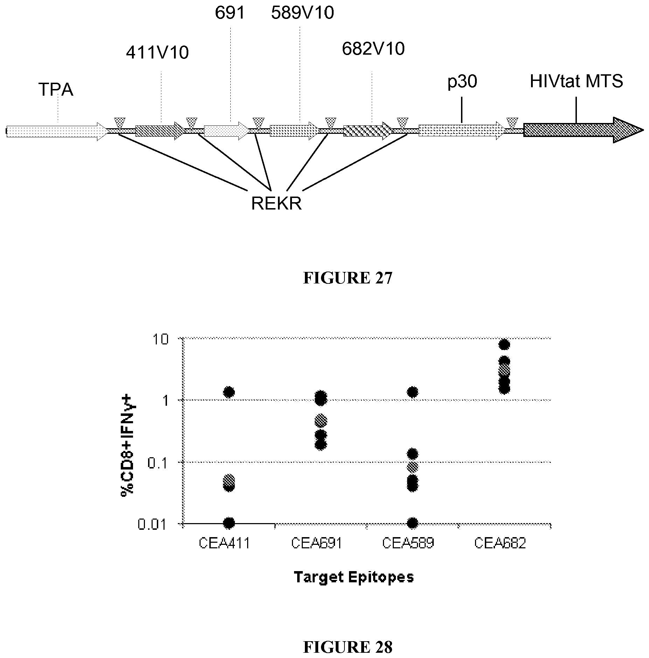

The LTB functional element in the minigenes described in this invention allows the translated and secreted product to enter MHC Class I processing pathway. Another method known in the art to direct exogenous peptides for presentation to CD8+ T-cells is to include a membrane-translocating sequence such as HIVtat peptide AAARKKRRQRRRR (SEQ ID NO:49) (see Kim et al, J. Immunol, 159, 1666-1668, 1997). We investigated whether replacing LTB with an MTS could enhance CD8+ T-cell response against the individual epitopes. Four HHD/CEA mice were immunized with a minigene containing HIVtat MTS, see Figure 27. The immunization protocol was the same as in Examples 17 and 20.

The immune response elicited by the construct is shown in Figure 28 and was lower compared to that elicited by an LTB-containing minigene, especially against 691 and 589 epitope (compare with Figure 21 in Example 21).

## Claims

1. A minigene coding for a protein comprising:
(a) a human tissue plasminogen signal peptide;
(b) at least one T-cell epitope; and
(c) an E. coli heat labile enterotoxin B subunit; wherein
(d) the at least one T-cell epitope is linked to the rest of the minigene, and to any other epitopes, by furin sensitive linkers having the sequence REKR.

2. A minigene according to claim 1 wherein the T-cell epitope is an immunogenic analogue of a naturally occurring epitope.

3. A minigene according to claim 1 or 2 wherein the T-cell epitope is from a microbial or tumour associated antigen or from the variable domain of an autoantibody.

4. A minigene according to any preceding claim wherein the T-cell epitope is restricted by an HLA allele.

5. A minigene according to any preceding claim comprising from two to twenty epitopes.

6. A minigene according to any preceding claim wherein the at least one T-cell epitope is from one or more of CEA, her-2/neu and hTERT.

7. A minigene according to any preceding claim having more than one T-cell epitope restricted by more than one HLA allele.

8. A minigene according to any preceding claim wherein the T-cell epitope is repeated.

9. A minigene according to any preceding claim comprising furin-sensitive linkers having at least two different sequences.

10. A minigene according to any preceding claim further comprising a T-helper epitope.

11. A minigene according to claim 10 wherein the T-helper epitope is tetanus toxin-derived universal helper peptide p2 or p30.

12. A pharmaceutical composition comprising a minigene according to any one of claims 1 to 11 and a pharmaceutically acceptable adjustment.

13. A minigene according to any one of claims 1 to 11 for use in a method of treatment of the human or animal body by therapy or prophylaxis.

14. A minigene for use according to claim 13 for treating or preventing cancer, an infectious disease or an autoimmune disease.

## Patentansprüche

1. Ein Minigen, das für ein Protein kodiert, umfassend:
(a) ein menschliches Gewebeplasminogen-Signalpeptid,
(b) wenigstens ein T-Zell-Epitop und
(c) eine hitzelabile Enterotoxin-B-Untereinheit von E. coli, wobei
(d) das wenigstens eine T-Zell-Epitop durch Furin-sensitive Linker mit der Sequenz REKR an den Rest des Minigens und an sonstige Epitope geknüpft ist.

2. Ein Minigen gemäß Anspruch 1, wobei das T-Zell-Epitop ein immunogenes Analogon eines natürlich vorkommenden Epitops ist.

3. Ein Minigen gemäß Anspruch 1 oder 2, wobei das T-Zell-Epitop von einem mikrobiellen oder tumor-assoziierten Antigen oder von der variablen Domäne eines Autoantikörpers stammt.

4. Ein Minigen gemäß einem vorhergehenden Anspruch, wobei das T-Zell-Epitop durch ein HLA-Allel beschränkt wird.

5. Ein Minigen gemäß einem vorhergehenden Anspruch, umfassend zwei bis zwanzig Epitope.

6. Ein Minigen gemäß einem vorhergehenden Anspruch, wobei das wenigstens eine T-Zell-Epitop von einem oder mehreren CEA, Her-2/neu und hTERT stammt.

7. Ein Minigen gemäß einem vorhergehenden Anspruch mit mehr als einem T-Zell-Epitop, beschränkt durch mehr als ein HLA-Allel.

8. Ein Minigen gemäß einem vorhergehenden Anspruch, wobei das T-Zell-Epitop wiederholt wird.

9. Ein Minigen gemäß einem vorhergehenden Anspruch, umfassend Furin-sensitive Linker mit wenigstens zwei unterschiedlichen Sequenzen.

10. Ein Minigen gemäß einem vorhergehenden Anspruch, ferner umfassend ein T-Helfer-Epitop.

11. Ein Minigen gemäß Anspruch 10, wobei das T-Helfer-Epitop ein von Tetanus-Toxin-hergeleitetes universales Helfer-Peptid p2 oder p30 ist.

12. Eine pharmazeutische Zusammensetzung, umfassend ein Minigen gemäß einem der Ansprüche 1 bis 11 und eine pharmazeutisch annehmbare Anpassung.

13. Ein Minigen gemäß einem der Ansprüche 1 bis 11 zur Verwendung bei einem Verfahren zur Behandlung des Menschen- oder Tierkörpers durch Therapie oder Prophylaxe.

14. Ein Minigen zur Verwendung gemäß Anspruch 13 zur Behandlung oder Prävention von Krebs, einer Infektionskrankheit oder einer Autoimmunerkrankung.

## Revendications

1. Minigène codant pour une protéine comprenant:
(a) un peptide signal du plasminogène tissulaire humain;
(b) au moins un épitope de lymphocytes T; et
(c) une sous-unité d'entérotoxine B labile à la chaleur de *E. coli;* dans lequel:
(d) le au moins un épitope de lymphocytes T est lié au reste du minigène, et à n'importe quels autres épitopes, par des liants sensibles à la furine possédant la séquence REKR.

2. Minigène selon la revendication 1, dans lequel l'épitope de lymphocytes T est un analogue immunogénique d'un épitope d'origine naturelle.

3. Minigène selon la revendication 1 ou 2, dans lequel l'épitope de lymphocytes T est issu d'un antigène microbien ou associé à une tumeur ou issu du domaine variable d'un auto-anticorps.

4. Minigène selon l'une quelconque des revendications précédentes, dans lequel l'épitope de lymphocytes T est restreint par un allèle HLA.

5. Minigène selon l'une quelconque des revendications précédentes comprenant de deux à vingt épitopes.

6. Minigène selon l'une quelconque des revendications précédentes, dans lequel le au moins un épitope de lymphocytes T est issu d'un ou de plusieurs parmi CEA, her-2/neu et hTERT.

7. Minigène selon l'une quelconque des revendications précédentes possédant plus d'un épitope de lymphocytes T restreints par plus d'un allèle HLA.

8. Minigène selon l'une quelconque des revendications précédentes, dans lequel l'épitope de lymphocytes T est répété.

9. Minigène selon l'une quelconque des revendications précédentes comprenant des liants sensibles à la furine possédant au moins deux séquences différentes.

10. Minigène selon l'une quelconque des revendications précédentes comprenant en outre un épitope de lymphocytes T auxiliaires.

11. Minigène selon la revendication 10, dans lequel l'épitope de lymphocytes T auxiliaires est un peptide auxiliaire universel dérivé de la toxine tétanique p2 ou p30.

12. Composition pharmaceutique comprenant un minigène selon l'une quelconque des revendications 1 à 11 et un ajustement pharmaceutiquement acceptable.

13. Minigène selon l'une quelconque des revendications 1 à 11 pour une utilisation dans une méthode de traitement du corps humain ou animal par thérapie ou prophylaxie.

14. Minigène pour une utilisation selon la revendication 13 pour le traitement ou la prévention d'un cancer, d'une maladie infectieuse ou d'une maladie auto-immune.
